# EUROPEAN PATENT APPLICATION

(11) **EP 2 801 573 A1**
(43) Date of publication of application: **12.11.2014**
(21) Application number: 13462001.2
(22) Date of filing: 09.05.2013
(51) Int. Cl.: C07D 401/14, C07D 405/14, C07D 413/14, C07D 401/08

(54) **Hydantoine derivatives as CD38 inhibitors**

(71) Applicant: SANOFI, 75008 Paris (FR)
(72) Inventor: Kapui, Zoltán, 1045 Budapest (HU); Vasas, Attila, 1045 Budapest (HU); Buzder-Lantos, Péter, 1045 Budapest (HU); Bátori, Sándor, 1045 Budapest (HU); Szabó, Tibor, 1045 Budapest (HU); Urbán-Szabó, Katalin, 1045 Budapest (HU); Ferenczy, György, 1045 Budapest (HU); Balogh, Mária, 1045 Budapest (HU)
(74) Representative: Tepfenhárt, Dóra Andrea

(57) **Abstract**

The present invention relates to compounds of formula I 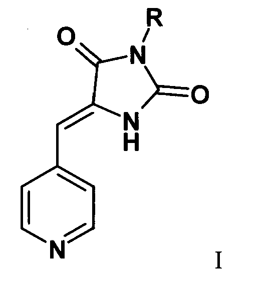 which have CD38 inhibitory activity and are useful as an active ingredient of a medicament for preventive and/or therapeutic treatment of inflammatory diseases.

## Description

The present invention relates to compounds that are useful as an active ingredient of a medicament for preventive and/or therapeutic treatment with CD38 inhibitory activity.

Chronic obstructive pulmonary diseases (COPD) is a major and increasing global health problem with enormous amount of expenditure of indirect/direct health-care costs. COPD now affects >10% of the world population over the age of 40 years, and the burden of disease is particularly high in developing countries. Currently the global economic burden is $15 billion, and it is anticipated that COPD will become the 3^{rd} leading cause of death worldwide by 2020. The greatest risk factor is cigarette smoke (some 80% of COPD patients). Current therapies are inadequate because no treatments reduce disease progression or mortality. The therapies are based mainly on anti-inflammatory drugs developed for asthma (e.g. corticosteroids) which are often ineffective in reducing the inflammation and airway lung function decline. In addition, bronchodilators and other agents used to address different features of the disease in which polypragmasy is the rule rather than the exception.

Inflammatory Bowel Disease (IBD) is a chronic, relapsing and remitting inflammatory disease of the GI tract. In Europe and North America, there are an estimated 2 million IBD patients.

A large unmet medical need exist for both diseases. In COPD, especially the need is critical for compounds that not only halt the destructive inflammation but reverse the progressive loss of cells and tissue leading to loss of alveolar structure in the lower airways. Further advantage would be if the compound had bronchodilatory activity or positive effect on airway hyper-responsiveness. In IBD, the unmet medical need is significant for oral therapeutics with novel mechanism of action (MOA) for treating non-responders to anti-TNFα antibody treatment.

CD38 is a type II trans-membrane glycoprotein of 45KDa, that is extensively expressed on cells of hematopoetic and non-hematopoetic linage, tissues and organs.

Its large, 257 aa long ectodomain displays both ectoenzyme activity and receptor function.

CD38 is a multi-functional enzyme, catalyzes the formation of both cyclic ADP-ribose and ADP-ribose products from NAD⁺ and hydrolyzes cyclic ADP-ribose to ADP-ribose. The corresponding GDP products are formed from NGD⁺. CD38 produces three Ca-mobilizing second messengers from NAD: cyclic ADP-ribose (cADPR), ADP-ribose (ADPR) and nicotinic acid adenine dinucleotide phosphate (NAADP). The cADPR is a cyclic nucleotide that activates intracellular Ca²⁺ release via types 2 and 3 ryanodine receptors and mobilizes the endoplasmic Ca²⁺ stores. ADPR (and cADPR) also evokes Ca²⁺ entry possibly through the activation of the oxidative stress responsive cation channel, TRPM2. NAADP forms in acidic condition, targets separate acidic Ca²⁺ stores, in acidic organelles like lysosomes.

cADPR and CD38 may play a crucial role in the human immune response and associated inflammatory physiology.

The cell surface ectoenzyme CD38 acts as a sensor of inflammation, tissue damage and necrosis, because its expression is increased in inflammatory conditions and its activity is highly regulated by substrate availability. In inflammatory conditions the extracellular level of NAD increases significantly up to µM range. The activated CD38 produces more cADPR, increases chemotactic response and cytokine production of inflammatory cells, whereby it leads to a more pronounced inflammation.

Its enzyme activity also senses the redox status of the cells and responding with the increased ratio of cADPR/ADPR. The decreased ratio of β-NAD/β-NADH and decreased GSH increases the production of cADPR. Finally activated CD38 increases the consequences of oxidative stress through Ca overload and TRPM2 channel activity.

Experimental data support, that inhibitors of CD38 have potential to modulate the Ca²⁺ dependent functions:
- Cell migration (neutrophils, monocytes, DC cells) and cytokine production in immunocytes (macrophages, monocytes, DC, T cells) and epithelial cells (A549)
- Contractility of airway smooth muscle cells and airway hyperreactivity (increased cADPR production sensitize smooth muscle contractility response to different agonists): CD38 KO mice have decreased AHR and in hASM cells, cytokines (TNFα, IFNγ, IL13) increase the expression of CD38 and smooth muscle contractility
- Oxidative stress induced damages: Mouse Embryonic Fibroblasts from CD38 KO mice are resistant to oxidative stress through the inhibition of intracellular Ca overload and ROS production

CD38 is a unique target with three separate mechanisms to address the main characteristic features of COPD: airway inflammation, airway hyper-reactivity and oxidative stress.

On the basis of the above results a potent CD38 inhibitor would alloy all the therapeutic features of anti-inflammatory, anti-oxidant and airway smooth muscle relaxant agents in one drug, with potential therapeutic benefit in COPD treatment. It would reduce the exacerbations (rate and severity), mitigate the severity of airway inflammation and improve the lung function and quality of life.

CD38 inhibitors may have potential activity in IBD treatment with new MoAs, where chronic inflammation and oxidative stress is an important factor in the aetiology of the disease (fig. 1.). A first-in-class oral CD38 inhibitor could be an effective drug in the threatening chronic inflammatory diseases, COPD and IBD. Am J Respir Cell Mol Biol. 32, pp 149-156, 2005; Biochem. Biphys. Res. Com. 346, pp 188-192, 2006; FEBS J. 272, pp 4590-4597, 2005; Leukemia Res. 25, pp 1-12, 2001; Am J Respir Cell Mol Biol. 31, pp 36-42, 2004; FASEB J. 17, pp 452-4, 2003; Am J Physiol Lung Cell Mol Physiol. 294, pp 378-85, 2008; Am J Physiol Lung Cell Mol Physiol. 292, pp 1385-95, 2007; FASEB J. 20, pp 1000-2, 2006; Am J Respir Cell Mol Biol. 40, pp 433-442, 2009.

Reduced allergen induced airway inflammation / Th2 cytokine production and mucus hyper-secretion were shown in CD38 knock-out mice (Am. J. Respir. Cell. Mol. Biol 40:433-442, 2009)

Airway smooth muscle cells (ASMCs) from asthmatic patients showed increased CD38 expression after TNF-□ stimulation compared with ASM cells derived from healthy individuals (Am J Physiol Lung Cell Mol Physiol 299:L879-L890, 2010)

CD38 knockout mice are protected against high-fat diet-induced obesity (FASEB J. 21:3629-3639, 2007). The mechanism of action supposed to be mediation of NAD level and SIRT1 activity (Curr Pharm Des. 2009; 15(1): 57-63.)

Using Streptozotocine - induced diabetic mice model, treatment with CD38 inhibitor ameliorated kidney hypertrophic responses (Am J Physiol Renal Physiol 296: F291-F297, 2009.)

It was demonstrated in a rat model, that CD38 inhibitor can reduce Angiotensine II-induced cardiac hypertrophy (Cardiovascular Research (2009) 81, 582-591)

CD38 knock-out mice showed decreases the cerebral ischemic injury and the persistent neurological deficit after three days of reperfusion in this murine temporary middle cerebral artery occlusion (tMCAO) model. On the other hand, an up-regulation of CD38 expression was observed in macrophages and CD8+ cells after focal cerebral ischemia in wildtype mice, whereas CD38 expression was unchanged in microglia. (PLoS ONE 6(5): e19046. 2011)

It also was demonstrated that CD38 expression was increased in peripheral blood mononuclear cells (PBMCs) at the early time points after ischemic stroke. (Neuroscience 160:394-401, 2009)

The above supports the potential activity of CD38 inhibitors in the treatment of asthma, allergy, obesity, metabolic syndrome, diabetic nephropathy, cardiac hypertrophy and cerebral ischemia.

We aimed to prepare new small molecular CD38 inhibitor compounds, which have strong inhibitory effect and are selective to the CD38 protein. We also aimed that the stability, bioavailability, metabolism, therapeutic index, toxicity and solubility of the new compounds allow their development into a drug substance. A further aim was that the compounds, due to their favourable enteric absorption, can be administered orally.

Thus, the inventors of the present invention have identified compounds represented by the following formula I possessing inhibitory activity against CD38 enzyme activity.

The present invention thus provides a compound of formula I 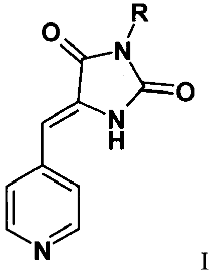 wherein
R represents a group of formula (II) 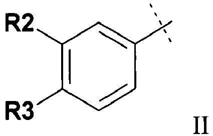 wherein R2 and R3 represent independently from each other H, halogen, cyano, C₁-C₄ alkyl, acetyl, C₁-C₄ halogenalkyl, C₁-C₄ alkyloxy, C₁-C₄ halogenalkoxy, C₁-C₄ alkylthio, C₁-C₄ alkylsulfinyl, C₁-C₄ alkylsulfonyl, -COOH, -(C₁-C₄ alkyl)-COOH, -NR4R5, wherein R4 and R5 are independently from each other H or C₁-C₄ alkyl; or -CO-NRxRy, wherein
Rx represents C₃-C₆ cycloalkyl or C₁-C₂ alkyl substituted with morpholinyl, and Ry represents H, or
Rx and Ry form together with the nitrogen attached a 5 or 6 membered heterocyclic ring optionally containing one further heteroatom selected from O and N and optionally substituted with one or more substituents selected from the group consisting of C₁-C₂ alkyl
and -COO-(C₁-C₄ alkyl); or
R represents C₁-C₆ alkyl optionally substituted with one or more substituents selected from the group consisting of -OH, -COOH, -COO-(C₁-C₄ alkyl), -NH-CO-CF₃, -CF₃, C₃-C₆ cycloalkyl, optionally substituted by -OH or -CH₂-OH; and a 5 or 6 membered heterocyclic ring optionally containing one or two heteroatom selected from O, N and S; or
R represents C₃-C₆ cycloalkyl optionally substituted with one or more substituents selected from the group consisting of OH and halogen; or
R represents phenyl-(C₁-C₂ alkyl), optionally substituted by one or more C₁-C₄ alkoxy; adamantyl, or pyridyl;
or a pharmaceutically acceptable salt or stereoisomer thereof or a pharmaceutically acceptable salt of the stereoisomer.

According to another aspect of the present invention, there is provided a process for the preparation of a compound of formula I or a pharmaceutically acceptable salt, stereoisomer or a pharmaceutically acceptable salt of the stereoisomer, comprising the steps according to the following reaction scheme:

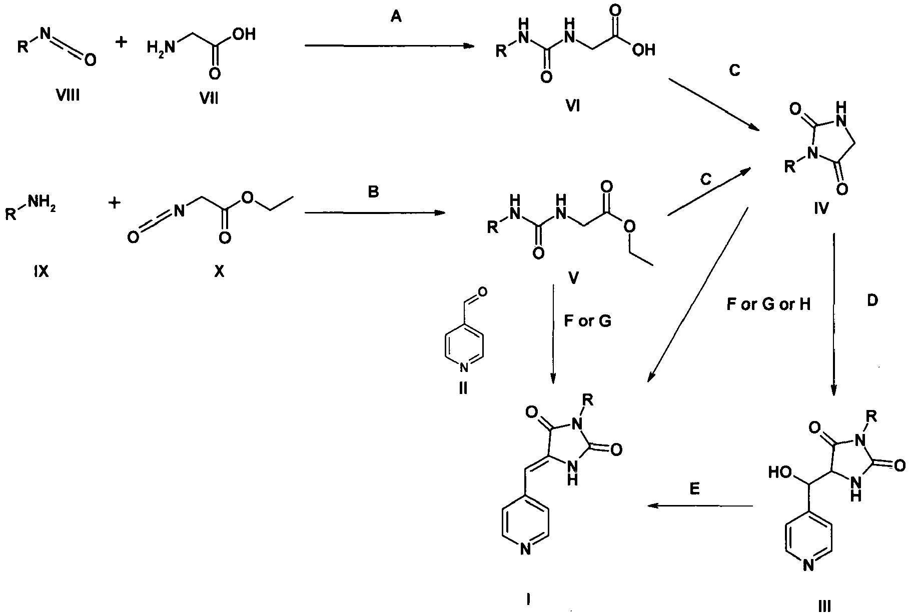

According to a further aspect the present invention is directed to a compound of formula I or a pharmaceutically acceptable salt or stereoisomer thereof or a pharmaceutically acceptable salt of the stereoisomer for use as a medicament.

According to another aspect of the present invention there is provided a pharmaceutical composition containing at least one compound of formula I or a pharmaceutically acceptable salt, stereoisomer or a pharmaceutically acceptable salt of the stereoisomer and at least one pharmaceutically acceptable excipient.

According to a further aspect the present invention is directed to the compounds of formula I or a pharmaceutically acceptable salt, stereoisomer or a pharmaceutically acceptable salt of the stereoisomer for use in the treatment or prevention of a disease or disorder selected from the group consisting of inflammatory diseases such as COPD, IBD, asthma, allergy, obesity, metabolic syndrome, diabetic nephropathy, cardiac hypertrophy and cerebral ischemia.

According to a specific aspect the invention is directed to the compounds of formula I or a pharmaceutically acceptable salt, stereoisomer or a pharmaceutically acceptable salt of the stereoisomer for use in the treatment or prevention of COPD or IBD.

In addition the present invention is directed to a method of treating inflammatory diseases such as COPD, IBD, asthma, allergy, obesity, metabolic syndrome, diabetic nephropathy, cardiac hypertrophy and cerebral ischemia comprising administering an effective amount of compounds of formula I or a pharmaceutically acceptable salt or stereoisomer thereof or a pharmaceutically acceptable salt of the stereoisomer to a patient in need thereof.

According to a specific aspect the present invention is directed to a method of treating COPD or IBD comprising administering an effective amount of compounds of formula I or a pharmaceutically acceptable salt or stereoisomer thereof or a pharmaceutically acceptable salt of the stereoisomer to a patient in need thereof.

As used above and throughout the description of the invention, the following terms, unless otherwise indicated, are to be understood to have the following meanings:

The C1-C6 alkyl, C1-C4 alkyl and C1-C2 alkyl group represents a straight or branched saturated hydrocarbon group having 1 to 6, 1 to 4 or 1 to 2 carbon atoms, respectively, for example, methyl group, ethyl group, n-propyl group, isopropyl group, n-butyl group, isobutyl group, sec-butyl group, tert-butyl group, the pentyl groups, hexyl groups, and the like.

The C₁-C₄ halogenalkyl group represents a C₁-C₄ alkyl group as defined above and substituted by one or more halogen atom, such as fluorine, chlorine, bromine and/or iodine atom, for example, fluoromethyl group, chloromethyl group, bromomethyl group, difluoromethyl group, dichloromethyl group, dibromomethyl group, trifluoromethyl group, trichloromethyl group, fluoroethyl group, chloroethyl group, bromoethyl group, difluoroethyl group, dichloroethyl group, fluoropropyl group, chloropropyl group, difluoropropyl group, dichloropropyl group, and the like.

The C₁-C₄ alkoxy group represents a C₁-C₄ alkyl group as defined above and attached through an oxygen atom, for example, methoxy group, ethoxy group, propoxy group, isopropoxy group, butoxy group, isobutoxy group, sec-butoxy group, tert-butoxy group, and the like.

The C₁-C₄ halogenalkyloxy group represents a C₁-C₄ alkyloxy group as defined above and substituted by one or more halogen atom, such as fluorine, chlorine, bromine and/or iodine atom, for example, fluoromethoxy group, chloromethoxy group, bromomethoxy group, difluoromethoxy group, dichloromethoxy group, dibromomethoxy group, trifluoromethoxy group, trichloromethoxy group, fluoroethoxy group, chloroethoxy group, bromoethoxy group, difluoroethoxy group, dichloroethoxy group, fluoropropoxy group, chloropropoxy group, difluoropropoxy group, dichloropropoxy group, and the like.

The C₁-C₄ alkylthio group represents a C₁-C₄ alkyl group as defined above and attached trough a sulphur atom, for example, methylthio group, ethylthio group, n-propylthio group, isopropylthio group, n-butylthio group, isobutylthio group, sec-butylthio group, tert-butylthio group, and the like.

The C₁-C₄ alkylsulfinyl group represents a C₁-C₄ alkyl group as defined above and attached trough a sulfinyl group (-SO-), for example, methylsulfinyl group, ethylsulfinyl group, n-propylsulfinyl group, isopropylsulfinyl group, n-butylsulfinyl group, isobutylsulfinyl group, sec-butylsulfinyl group, tert-butylsulfinyl group, and the like.

The C₁-C₄ alkylsulfonyl group represents a C₁-C₄ alkyl group as defined above and attached trough a sulfonyl group (-SO₂-), for example, methylsulfonyl group, ethylsulfonyl group, n-propylsulfonyl group, isopropylsulfonyl group, n-butylsulfonyl group, isobutylsulfonyl group, sec-butylsulfonyl group, tert-butylsulfonyl group, and the like.

The C₃-C₆ cycloalkyl group represents a cyclic hydrocarbon group having 3 to 6 carbon atoms, for example, cyclopropyl group, cyclobutyl group, cyclopentyl group, cyclohexyl group, and the like.

The 5 or 6 membered heterocyclic ring represents a saturated hydrocarbon ring containing one or more heteroatom selected from O, N and S, for example pyrrolidinyl ring, pyperidinyl ring, tetrahydrofuranyl ring, tetrahydropyranylring, morpholinyl ring, and the like.

The halogen atom represents a fluorine, chlorine, bromine or iodine atom, specifically fluorine atom.

By salts of the compounds of the formula I we mean salts formed with inorganic and organic acids. Preferred salts are those given with pharmaceutically acceptable acids as for instance hydrochloric acid, and the like. The salts formed during purification or isolation are also subject of the invention.

By stereoisomers of the compounds of the formula I we mean all isomers of the individual molecules that differ only in the orientation of their atoms in space.

Typically it includes mirror image optical isomers that are usually formed due to at least one asymmetric center (enantiomers or diastereoisomers). These enantiomers and diastereoisomers, as well as their mixtures, including the racemates, are also subjects of the invention.

Also compounds of the formula I may exist in the form of geometric isomers (cis/trans or E/Z isomers). These geometric isomers are also subject of the invention.

One of the embodiments of the present invention includes a compound of formula I, wherein
R represents a group of formula II
wherein
R2 and R3 represent independently from each other H, halogen, cyano, C₁-C₄ alkyl, acetyl, trifluoromethyl, methoxy, trifluoromethoxy, methylthio, methylsulfinyl, methylsulfonyl, -COOH,
- CH₂-COOH, dimethylamino; or -CO-NRxRy, wherein
Rx represents C₃-C₆ cycloalkyl or morpholinyl-ethyl, and Ry represents H, or
Rx and Ry form together with the nitrogen attached a pyrrolidinyl, a pyperidinyl or a morpholinyl ring optionally substituted with one or two substituents selected from the group
consisting of methyl and ethoxy-carbonyl; or
R represents C₁-C₆ alkyl optionally substituted with one or more substituents selected from the group consisting of -OH, -COOH, -COO-(C₁-C₂ alkyl), -NH-CO-CF₃, -CF₃, C₃-C₆ cycloalkyl, optionally substituted by -OH or -CH₂-OH; and tetrahydrofuranyl, tetrahydropyranyl, piperidinyl, or morpholinyl; or
R represents C₄-C₆ cycloalkyl optionally substituted with one or more substituents selected from the group consisting of -OH and fluorine; or
R represents phenyl-(C₁-C₂ alkyl), optionally substituted by two C₁-C₄ alkoxy; adamantyl, or pyridyl;
or a pharmaceutically acceptable salt or stereoisomer thereof or a pharmaceutically acceptable salt of the stereoisomer.

Another embodiment of the present invention includes a compound of formula I, wherein R represents a group of formula II
wherein
R2 and R3 represent independently from each other H, halogen, cyano, methyl, ethyl, propyl, isopropyl, *tert*-butyl, butyl, acetyl, trifluoromethyl, methoxy, trifluoromethoxy, methylthio, methylsulfinyl, methylsulfonyl, -COOH, -CH₂-COOH, dimethylamino; or -CO-NRxRy, wherein Rx represents C₃-C₆ cycloalkyl or morpholinyl-ethyl, and Ry represents H, or
Rx and Ry form together with the nitrogen attached a pyrrolidinyl, a pyperidinyl or a morpholinyl ring optionally substituted with two methyl or one ethoxy-carbonyl; or
R represents C₁-C₆ alkyl optionally substituted with one substituent selected from the group consisting of -OH, -CF₃, C₃-C₆ cycloalkyl, optionally substituted by -OH or -CH₂-OH; tetrahydrofuranyl and tetrahydropyranyl; or
R represents cyclobutyl substituted with one or two fluorine; or
R represents phenyl-(C₁-C₂ alkyl) or pyridyl;
or a pharmaceutically acceptable salt or stereoisomer thereof or a pharmaceutically acceptable salt of the stereoisomer.

Another embodiment of the present invention includes a compound of formula I, wherein R represents a group of formula II
wherein
R2 and R3 represent independently from each other H, halogen, cyano, C₁-C₄ alkyl, acetyl, trifluoromethyl, methoxy, trifluoromethoxy, methylthio, methylsulfinyl, methylsulfonyl, -COOH,
-CH₂-COOH, dimethylamino; or -CO-NRxRy, wherein
Rx represents C₃-C₆ cycloalkyl or morpholinyl-ethyl, and Ry represents H, or
Rx and Ry form together with the nitrogen attached a pyrrolidinyl, a pyperidinyl or a morpholinyl ring optionally substituted with one or two substituents selected from the group consisting of methyl and ethoxy-carbonyl,
or a pharmaceutically acceptable salt or stereoisomer thereof or a pharmaceutically acceptable salt of the stereoisomer.
wherein R represents C₁-C₆ alkyl optionally substituted with one or more substituents selected from the group consisting of OH, COOH, COO-(C₁-C₂ alkyl), NH-CO-CF₃, CF₃, C₃-C₆ cycloalkyl, optionally substituted by OH or CH₂-OH; tetrahydrofuranyl, tertahydropyranyl, morpholinyl and piperidinyl, or a pharmaceutically acceptable salt or stereoisomer thereof or a pharmaceutically acceptable salt of the stereoisomer.

Another embodiment of the present invention includes a compound of formula I,
wherein
R2 and R3 represent independently from each other H, halogen, cyano, methyl, ethyl, propyl, isopropyl, tertbutyl, butyl, acetyl, trifluoromethyl, methoxy, trifluoromethoxy, methylthio, methylsulfinyl, methylsulfonyl, -COOH, -CH₂-COOH, dimethylamino; or -CO-NRxRy, wherein Rx represents C₃-C₆ cycloalkyl or morpholinyl-ethyl, and Ry represents H, or
Rx and Ry form together with the nitrogen attached a pyrrolidinyl, a pyperidinyl or a morpholinyl ring optionally substituted with two methyl or one ethoxy-carbonyl,
or a pharmaceutically acceptable salt or stereoisomer thereof or a pharmaceutically acceptable salt of the stereoisomer.

Another embodiment of the present invention includes a compound of formula I
wherein
R represents C₁-C₆ alkyl optionally substituted with one or more substituents selected from the group consisting of -OH, -COOH, -COO-(C₁-C₂ alkyl), -NH-CO-CF₃, -CF₃, C₃-C₆ cycloalkyl, optionally substituted by -OH or -CH₂-OH; tetrahydrofuranyl, tertahydropyranyl, morpholinyl and piperidinyl,
or a pharmaceutically acceptable salt or stereoisomer thereof or a pharmaceutically acceptable salt of the stereoisomer.

Another embodiment of the present invention includes a compound of formula I,
wherein
R represents C₁-C₆ alkyl optionally substituted with one substituent selected from the group consisting of -OH, -CF₃, C₃-C₆ cycloalkyl, optionally substituted by -OH or -CH₂-OH; tetrahydrofuranyl and tertahydropyranyl;
or a pharmaceutically acceptable salt or stereoisomer thereof or a pharmaceutically acceptable salt of the stereoisomer.

Another specific embodiment of the present invention includes a compound of formula I, wherein
R represents C₁-C₆ alkyl optionally substituted with one substituent selected from the group consisting of -OH and C₃-C₆ cycloalkyl, optionally substituted by -OH or -CH₂-OH;
or a pharmaceutically acceptable salt or stereoisomer thereof or a pharmaceutically acceptable salt of the stereoisomer.

Another more specific embodiment of the present invention includes a compound of formula I,
wherein
R represents C₁-C₄ alkyl substituted with one substituent selected from the group consisting of - OH and C₃-C₆ cycloalkyl substituted with -OH or -CH₂-OH;
or a pharmaceutically acceptable salt or stereoisomer thereof or a pharmaceutically acceptable salt of the stereoisomer.

Another embodiment of the present invention includes a compound of formula I,
wherein
R represents C₄-C₆ cycloalkyl optionally substituted with one or more substituents selected from the group consisting of -OH and fluorine,
or a pharmaceutically acceptable salt or stereoisomer thereof or a pharmaceutically acceptable salt of the stereoisomer.

Another embodiment of the present invention includes a compound of formula I,
wherein
R represents C₄ cycloalkyl substituted with one or two fluorine.
or a pharmaceutically acceptable salt or stereoisomer thereof or a pharmaceutically acceptable salt of the stereoisomer.

Another embodiment of the present invention includes a compound of formula I,
wherein
R represents phenyl-(C₁-C₂ alkyl), optionally substituted by one or two C₁-C₄ alkoxy; adamantyl, or pyridyl
or a pharmaceutically acceptable salt or stereoisomer thereof or a pharmaceutically acceptable salt of the stereoisomer.

Another embodiment of the present invention includes a compound of formula I, wherein
R represents phenyl-(C₁-C₂ alkyl) or pyridyl or a pharmaceutically acceptable salt or stereoisomer thereof or a pharmaceutically acceptable salt of the stereoisomer.

Another embodiment of the present invention includes a compound of formula I in its Z isomeric form.

Particularly compounds of the present invention represented by formula I include compounds selected from the group consisting of:
1, 3-Phenyl-5-[1-pyridin-4-yl-meth-(*Z*)-ylidene]-imidazolidine-2,4-dione.HCl salt
2, 3-(4-Chloro-phenyl)-5-[1-pyridin-4-yl-meth-(*Z*)-ylidene]-imidazolidine-2,4-dione
3, 5-[1-Pyridin-4-yl-meth-(*Z*)-ylidene]-3-m-tolyl-imidazolidine-2,4-dione
4, 3-(4-Ethyl-phenyl)-5-[1-pyridin-4-yl-meth-(Z)-ylidene]-imidazolidine-2,4-dione
5, 3-(3-Chloro-phenyl)-5-[1-pyridin-4-yl-meth-(*Z*)-ylidene]-imidazolidine-2,4-dione HCl-salt
6, 3-(3,4-Dichloro-phenyl)-5-[1-pyridin-4-yl-meth-(*Z*)-ylidene]-imidazolidine-2,4-dione
7, 3-Benzyl-5-[1-pyridin-4-yl-meth-(*Z*)-ylidene]-imidazolidine-2,4-dione
8, 3-(4-Methylsulfanyl-phenyl)-5-[1-pyridin-4-yl-meth-(*Z*)-ylidene]-imidazolidine-2,4-dione
9, 3-Cyclohexyl-5-[1-pyridin-4-yl-meth-(*Z*)-ylidene]-imidazolidine-2,4-dione
10, 3-(3,4- Difluoro-phenyl)-5-[1-pyridin-4-yl-meth-(*Z*)-ylidene]-imidazolidine-2,4-dione
11, 4-{2,5-Dioxo-4-[1-pyridin-4-yl-meth-(*Z*)-ylidene]-imidazolidin-1-yl} -benzonitrile
12, 3-(4-Fluoro-phenyl)-5-[1-pyridin-4-yl-meth-(2)-ylidene]-imidazolidine-2,4-dione
13, 3-(3-Acetyl-phenyl)-5-[1-pyridin-4-yl-meth-(*Z*)-ylidene]-imidazolidine-2,4-dione
14,3-(4-Dimethylamino-phenyl)-5-[1-pyridin-4-yl-meth-(*Z*)-ylidene]-imidazolidine-2,4-dione
15, 4-{2,5-Dioxo-4-[1-pyridin-4-yl-meth-(*Z*)-ylidene]-imidazolidin-l-yl}-benzoic acid
16, 5-[1-Pyridin-4-yl-meth-(*Z*)-ylidene]-3-(3-triftuoromethyl-phenyl)-imidazolidine-2,4-dione
17,3-(4-Methoxy-phenyl)-5-[1-pyridin-4-yl-meth-(*Z*)-ylidene]-imidazolidine-2,4-dione
18,3-Phenethyl-5-[1-pyridin-4-yl-meth-(*Z*)-ylidene]-imidazolidine-2,4-dione
19, 5-[1-Pyndin-4-yl-meth-(*Z*)-ylidene]-3-p-totyl-imidazolidine-2,4-dione
20, 3-(4-Bromo-phenyl)-5-[1-pyridin-4-yl-meth-(*Z*)-ylidene]-imidazolidine-2,4-dione
21, 3-Isopropyl-5-[1-pyridin-4-yl-meth-(*Z*)-ylidene]-imidazolidine-2,4-dione
22, 3-Hexyl-5-[1-pyridin-4-yl-meth-(*Z*)-ylidene]-imidazotidine-2,4-dione
23, 3-(4-Acetyl-phenyl)-5-[1-pyridin-4-yl-meth-(*Z*)-ylidene]-imidazolidine-2,4-dione
24, 3-(4-Butyl-phenyl)-5-[1-pyridin-4-yl-meth-(*Z*)-ylidene]-imidazolidine-2,4-dione
25, 3-(4-Methanesulfinyl-phenyl)-5-[1-pyridin-4-yl-meth-(*Z*)-ylidene]-imidazolidine-2,4-dione
26, 3-(4-Methanesulfonyl-phenyl)-5-[1-pyridin-4-yl-meth-(*Z*)-ylidene]-imidazolidine-2,4-dione
27, 3-{2,5-Dioxo-4-[1-pyridin-4-yl-meth-(*Z*)-ylidene]-imidazolidin-1-yl}-benzoic acid
28, 3-[3-(Morpholine-4-carbonyl)-phenyl]-5-[1-pyridin-4-yl-meth-(*Z*)-ylidene]-imidazolidine-2,4-dione
29, 3-[4-(Morpholine-4-carbonyl)-phenyl]-5-[1-pyridin-4-yl-meth-(*Z*)-ylidene]-imidazolidine-2,4-dione
30, 5-[1-Pyridin-4-yl-meth-(*Z*)-ylidene]-3-(4-trifluoromethoxy-phenyl)-imidazolidine-2,4-dione
31, 5-[1-Pyridin-4-yl-meth-(*Z*)-ylidene]-3-(4-trifluoromethyl-phenyl)-imidazolidine-2,4-dione
32, (4-{2,5-Dioxo-4-[1-pyndin-4-yl-meth-(*Z*)-ylidene]-imidazolidin-1-yl}-phenyl)-acetic acid
33, 3-(4-Iodo-phenyl)-5-[1-pyridin-4-yl-meth-(*Z*)-ylidene]-imidazolidine-2,4-dione
34, 3-{2,5-Dioxo-4-[1-pyridin-4-yl-meth-(*Z*)-ylidene]-imidazolidin-1-yl}-N-(2-morpholin-4-yl-ethyl)-benzamide
35, 3-(4-tert-Butyl-phenyl)-5-[1-pyridin-4-yl-meth-(*Z*)-ylidene]-imidazolidine-2,4-dione
36, 3-[2-(3,4-Diethoxy-phenyl)-ethyl]-5-[1-pyridin-4-yl-meth-(*Z*)-ylidene]-imidazolidine-2,4-dione
37, 5-[1-Pyridin-4-yl-meth-(*Z*)-ylidene]-3-(2-pyrrolidin-1-yl-ethyl)-imidazolidine-2,4-dione
38, 3-Adamantan-1-yl-5-[1-pyridin-4-yl-meth-(*Z*)-ylidene]-imidazolidine-2,4-dione
39, 3-Pyridin-3-yl-5-[1-pyridin-4-yl-meth-(*Z*)-ylidene]-imidazolidine-2,4-dione
40, 3-(4-Hydroxy-cyclohexyl)-5-[1-pyridin-4-yl-meth-(*Z*)-ylidene]-imidazolidine-2,4-dione
41, 3-Propyl-5-[1-pyridin-4-yl-meth-(*Z*)-ylidene]-imidazolidine-2,4-dione
42, 3-sec-Butyl-5-[1-pyridin-4-yl-meth-(*Z*)-ylidene]-imidazolidine-2,4-dione
43, {2,5-Dioxo-4-[1-pyridin-4-yl-meth-(*Z*)-ylidene]-imidazolidin-1-yl}-acetic acid ethyl ester
44, 3-(3-Morpholin-4-yl-propyl)-5-[1-pyridin-4-yl-meth-(*Z*)-ylidene]-imidazolidine-2,4-dione
45, 3-[4-((2R,6S)-2,6-Dimethyl-morpholine-4-carbonyl)-phenyl]-5-[1-pyridin-4-yl-meth-(Z)-ylidene]-imidazolidine-2,4-dione
46, {2,5-Dioxo-4-[1-pyridin-4-yl-meth-(*Z*)-ylidene]-imidazolidin-1-yl}-acetic acid
47, 3-[4-(4-Methyl-piperidine-1-carbonyl)-phenyl]-5-[1-pyridin-4-yl-meth-(*Z*)-ylidene]-imidazolidine-2,4-dione
48, 5-[1-Pyridin-4-yl-meth-(*Z*)-ylidene]-3-[4-(pyrrolidine-1-carbonyl)-phenyl]-imidazolidine-2,4-dione
49, N-Cyclopentyl-4-{2,5-dioxo-4-[1-pyridm-4-yl-meth-(*Z*)-ylidene]-imidazolidm-1-yl}-benzamide
50, N-Cyclopropyl-4-{2,5-dioxo-4-[1-pyridin-4-yl-meth-(*Z*)-ylidene]-imidazolidin-1-yl}-benzamide
51, N-Cyclohexyl-4-{2,5-dioxo-4-[1-pyndin-4-yl-meth-(*Z*)-ylidene]-imidazolidin-1-yl}-benzamide
52, 3-(3-Hydroxy-2,2-dimethyl-propyl)-5-[1-pyridin-4-yl-meth-(*Z*)-ylidene]-imidazolidine-2,4-dione
53, 3-(3,3-Difluoro-cyclobutyl)-5-[1-pyridin-4-yl-meth-(*Z*)-ylidene]-imidazolidine-2,4-dione
54, 1-(4-{2,5-Dioxo-4-[1-pyridin-4-yl-meth-(*Z*)-ylidene]-imidazotidin-l-yl}-benzoyl)-piperidine-4-carboxylic acid ethyl ester
55, 3-(1-Hydroxy-cyclohexylmethyl)-5-[1-pyridin-4-yl-meth-(*Z*)-ylidene]-imidazotidine-2,4-dione
56, 5-[1-Pyridin-4-yl-meth-(*Z*)-ylidene]-3-(tetrahydro-pyran-4-ylmethyl)-imidazolidine-2,4-dione
5 7, 3 -(3-Hydroxy-3-methyl-butyl)-5-[1-pyridin-4-yl-meth-(*Z*)-ylidene] -imidazolidine-2,4-dione
58, 3-(1-Hydroxy-cyclopentylmethyl)-5-[1-pyridin-4-yl-meth-(*Z*)-ylidene]-imidazolidine-2,4-dione
59, 3-(1-Hydroxy-cyclobutylmethyl)-5-[1-pyridin-4-yl-meth-(*Z*)-ylidene]-imidazolidine-2,4-dione
60, 5-[1-Pyridin-4-yl-meth-(*Z*)-ylidene]-3-(tetrahydro-furan-2-ylmethyl)-imidazolidine-2,4-dione
61, 3-(2-Hydroxy-2-methyl-propyl)-5-[1-pyridin-4-yl-meth-(*Z*)-ylidene]-imidazolidine-2,4-dione
62, 5-[1-Pyridin-4-yl-meth-(*Z*)-ylidene]-3-(2,2,2-trifluoro-ethyl)-imidazolidine-2,4-dione
63, 3-(1-Hydroxymethyl-cyclobutylmethyl)-5-[1-pyridin-4-yl-meth-(*Z*)-ylidene]-imidazolidine-2,4-dione
64, 3-(1-Hydroxymethyl-cyclopentylmethyl)-5-[1-pyridin-4-yl-meth-(*Z*)-ylidene]-imidazolidine-2,4-dione
65, N-(2-{2,5-Dioxo-4-[1-pyridin-4-yl-meth-(*Z*)-ylidene]-imidazolidin-1-yl}-ethyl)-2,2,2-trifluoro-acetamide
66, 3-(*trans*-4-Hydroxy-cyclohexylmethyl)-5-[1-pyridin-4-yl-meth-(*Z*)-ylidene]-imidazolidine-2,4-dione
or a pharmaceutically acceptable salt, stereoisomer or a pharmaceutically acceptable salt of the stereoisomer thereof.

### General procedures:

Starting materials used in the synthesis are known in the prior art and are obtained from chemical vendors such as Aldrich, Acros, Sigma, Fluka, Lancaster and others.

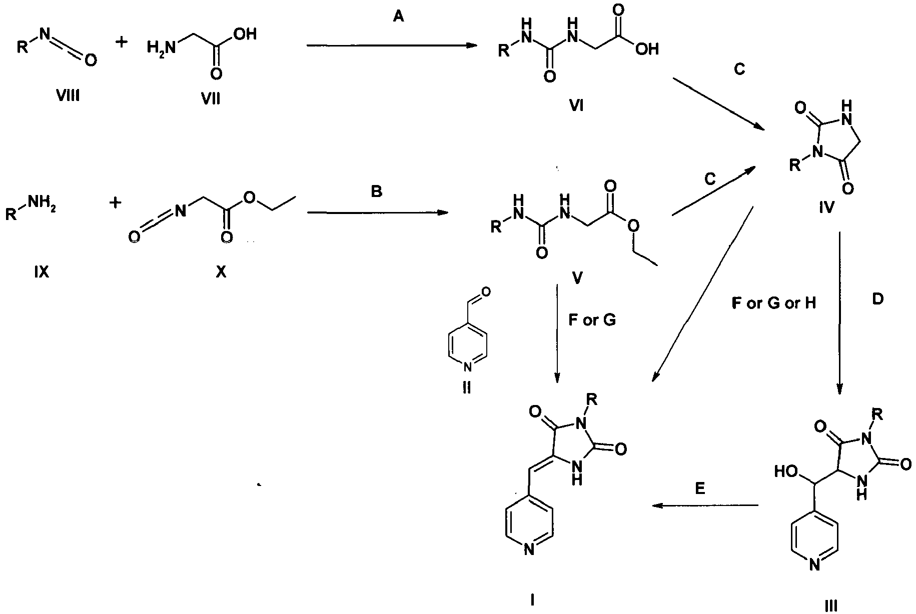

Conditions: **A:** H₂O / NaOH /50°C 1-2 h; **B:** CH₂Cl₂ / RT / 1-2 h; **C:** 1:1 HCl:H₂O / 120°C / 1-4 h; **D:** Et(iPrN)₂N or Et₃N / EtOH; **E:** AcOH / reflux; **F:** pyridine-4-carboxaldehyde, mesitylene or DMF, 180°C; **G:** pyridine-4-carboxaldehyde, DIPEA / CEM MW; **H:** pyridine-4-carboxaldehyde, Et₂NH or Et(iPrN)₂N / Synthewave 402 (Prolabo) MW; whereas R represents alkyl or aryl groups

In the first step **VI** carboxylic acids were synthesized in the reactions of the appropriate alkyl or aryl isocyanates **(VIII)** and glycine **(VII). V** carboxylic esters were synthesized in the reactions of the appropriate alkyl or arylamines **(IX)** and isocyanato ethylacetate **(X).** The cyclization of **V** or **VI** under acidic conditions gave the appropiate **IV** hydantoine. **I** substituted hydantoines are synthesized from **IV** hydantoine via condensation with pyridine-4-carboxaldehyde **(II)** under classic or microwave irradiated conditions in one or two step(s). **I** hydantoines are also available from **IX** amines via **V** esters as a one pot or a two steps synthesis.

As mentioned above the compounds of formula I or a pharmaceutically acceptable salt, stereoisomer or a pharmaceutically acceptable salt of the stereoisomer can be used as active ingredient of a medicament in the treatment or prevention of a disease or disorder selected from the group consisting of inflammatory diseases such as COPD, IBD, asthma, allergy, obesity, metabolic syndrome, diabetic nephropathy, cardiac hypertrophy and cerebral ischemia.

As the active ingredient of the medicament of the present invention, a substance may be used which is selected from the group consisting of the compound represented by the aforementioned formula I and pharmacologically acceptable salts, or stereoisomer thereof or a pharmaceutically acceptable salt of the stereoisomer. The substance, per se, may be administered as the medicament of the present invention, however, it is desirable to administer the medicament in a form of a pharmaceutical composition which comprises the aforementioned substance as an active ingredient and one or more pharmaceutical additives. As the active ingredient of the medicament of the present invention, two or more of the aforementioned substances may be used in combination. The type of pharmaceutical composition is not particularly limited, and the composition may be provided as any formulation for oral or parenteral administration. For example, the pharmaceutical composition may be formulated, for example, in the form of pharmaceutical compositions for oral administration such as granules, fine granules, powders, hard capsules, soft capsules, syrups, emulsions, suspensions, solutions and the like, or in the form of pharmaceutical compositions for parenteral administrations such as injections for intravenous, intramuscular, or subcutaneous administration, drip infusions, transdermal preparations, transmucosal preparations, nasal drops, inhalants, suppositories and the like. Injections or drip infusions may be prepared as powdery preparations such as in the form of lyophilized preparations, and may be used by dissolving just before use in an appropriate aqueous medium such as physiological saline.

Types of pharmaceutical additives used for the manufacture of the pharmaceutical composition, content ratios of the pharmaceutical additives relative to the active ingredient, and methods for preparing the pharmaceutical composition may be appropriately chosen by those skilled in the art. Inorganic or organic substances, or solid or liquid substances may be used as pharmaceutical additives. Generally, the pharmaceutical additives may be incorporated in a ratio ranging from 1 % by weight to 90% by weight based on the weight of an active ingredient.

The dose and frequency of administration of the medicament of the present invention are not particularly limited, and they may be appropriately chosen depending on conditions such as a purpose of preventive and/or therapeutic treatment, a type of a disease, the body weight or age of a patient, severity of a disease and the like. Generally, a daily dose for oral administration to an adult may be 0.01 to 1,000 mg (the weight of an active ingredient), and the dose may be administered once a day or several times a day as divided portions, or once in several days. When the medicament is used as an injection, administrations may preferably be performed continuously or intermittently in a daily dose of 0.001 to 100 mg (the weight of an active ingredient) to an adult.

The present invention will be explained more specifically with reference to the following examples, however, the scope of the present invention is not limited to these examples.

### Example I:

Synthesis of (Z)-3-(3,4-difluorophenyl)-5-(pyridin-4-ylmethylene)imidazolidine-2,4-dione

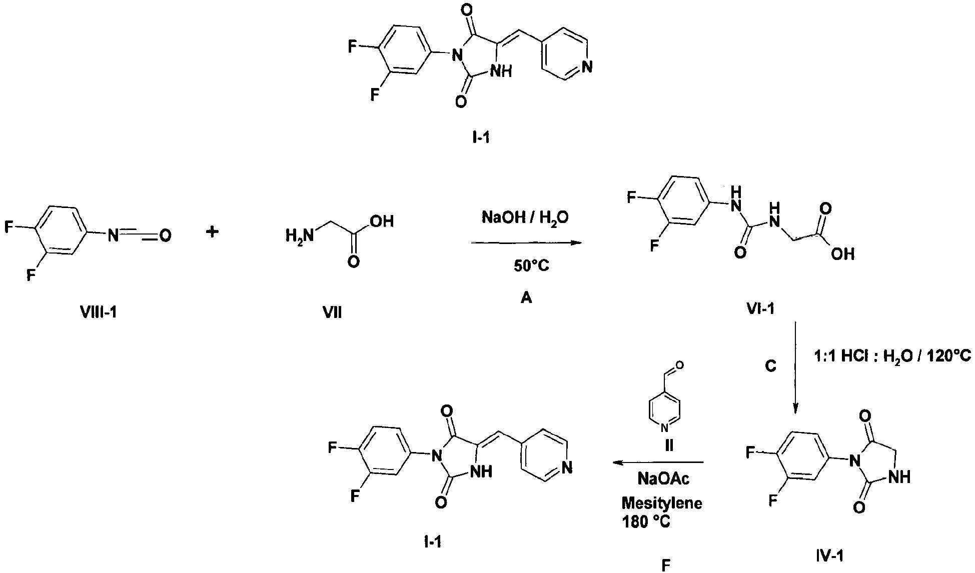

Route A: 0.75 g (0.01 mol) of glycine **(VII)** and 0.48 g (0.012 mol) sodium hydroxide were solved in 20 ml water and stirred for 2 minutes then 1.55 g (0.01 mol) 1,2-difluoro-4-isocyanatobenzene **(VIII-1)** was added. It was stirred for 45 minutes at 50°C. It was extracted with 50 ml ethanol at pH10. 1.5 ml of cc. HCl was added to the aqueous layer then it was cooled down. The white solid compound was filtered off and washed with 2x10 ml water. Resulted 1.637 g of 2-(3-(3,4-difluorophenyl)ureido)acetic acid **(VI-1)** with 71% yield as a white solid compound.

Route C: 1.53 g (6.65 mmol) 2-(3-(3,4-difluorophenyl)ureido)acetic acid **(VI-1)** was solved in 20% HCl and heated and stirred for 1 hour at 120 °C. It was cooled down to room temperature. The solid compound was filtered off and washed with 2x10 ml water. Resulted 1.1702 g of 3-(3,4-difluorophenyl)imidazolidine-2,4-dione **(IV-1)** with 83% yield as a white solid compound. Route F: 0.636 g (3 mmol) 3-(3,4-difluorophenyl)imidazolidine-2,4-dione **(IV-1),** 0.3211 g (3 mmol) pyridine4-carboxaldehyde **(II)** and 0.3689 g (4.5 mmol) sodium acetate were solved in 10 ml mesitylene and heated and stirred at 180°C for 3 days. It was cooled down to room temperature. The solid compound was filtered off then washed with 2x10 ml diisopropyl ether, 10 ml water and 10 ml methanol. Resulted 0.2626 g of (Z)-3-(3,4-difluorophenyl)-5-(pyridin-4-ylmethylene)imidazolidine-2,4-dione (I-1) with 29% yield as a beige solid compound.

### Example II:

Synthesis of (*Z*)-5-(pyridin-4-ylmethylene)-3-(4-(trifluoromethoxy)phenyl)imidazolidine-2,4-dione 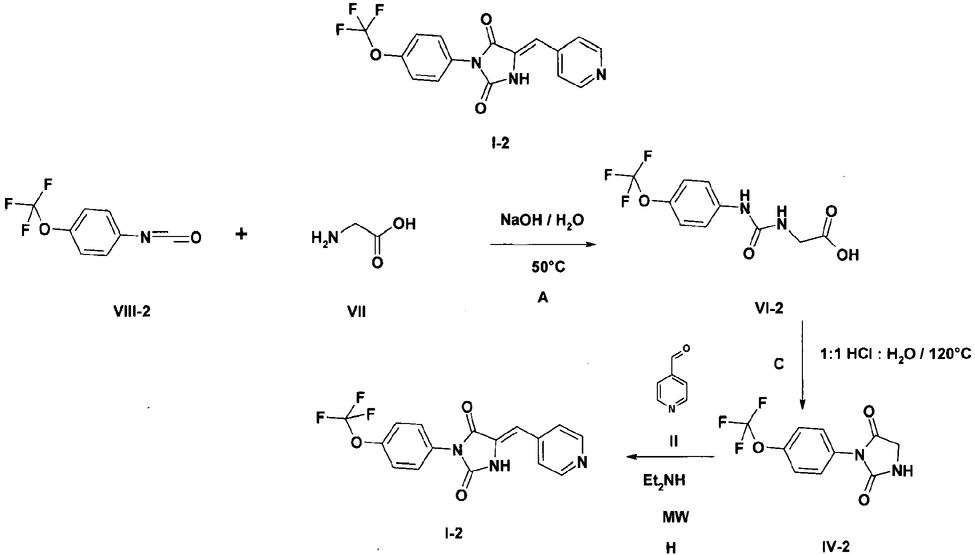 3-(4-(trifluoromethoxy)phenyl)imidazolidine-2,4-dione **(IV-2)** was synthesized *via* A and C route, as detailed in **Example I.**

Route H: 0.390 g (1.5 mmol) 3-(4-(trifluoromethoxy)phenyl)imidazolidine-2,4-dione **(IV-2),** 0.2 ml (2.12 mmol, d = 1.137) pyridine-4-carboxaldehyde **(II)** and 0.2 ml (1.93 mmol, d = 0.707) were reacted in Synthewave 402 (Prolabo) with 100% MW irradiation in 3.5 minutes. The temperature started at 26.4 °C and reached the final 129 °C at the end. It was washed with 5 ml methanol and 5 ml diisopropyl ether and filtered off. Resulted 0.1412 g (*Z*)-5-(pyridin-4-ylmethylene)-3-(4-(trifluoromethoxy)phenyl)imidazolidine-2,4-dione (**I-2**) with 26.9% yield as a beige solid compound.

### Example III:

Synthesis of (*Z*)-2-(4-(2,5-dioxo-4-(pyridin-4-ylmethylene)imidazolidin-1-yl)phenyl)acetic acid 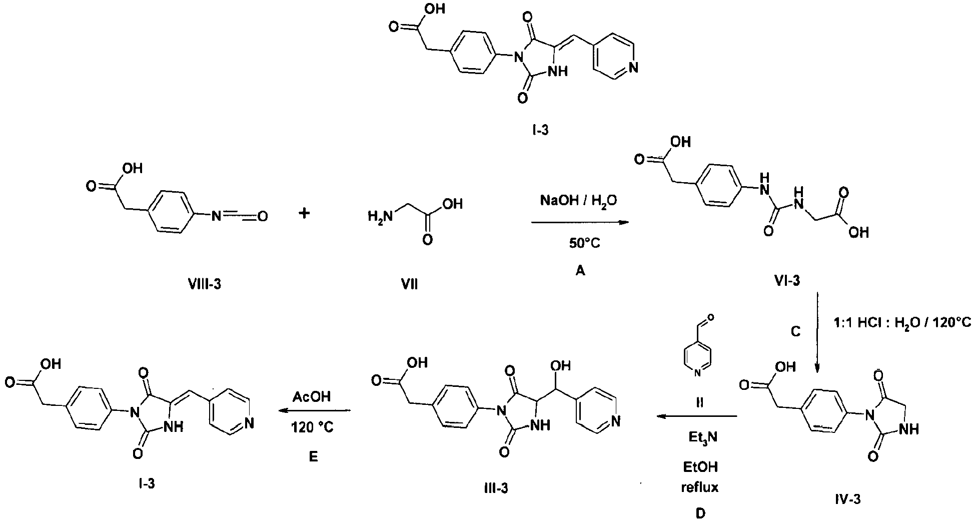 2-(4-(2,5-dioxoimidazolidin-1-yl)phenyl)acetic acid **(IV-3)** was synthesized in two steps *via* A and C route as detailed in **Example** I.

Route D, E: 0.327 g (1.39 mmol) 2-(4-(2,5-dioxoimidazolidin-1-yl)phenyl)acetic acid (IV-3), 0.15 ml (1.59 mmol, d = 1.137) pyridine-4-carboxaldehyde (**II**), 0.21 ml (1.5 mmol, d= 0.726) triethylamine were solved in 20 ml ethanol and heated and stirred at reflux temperature for 3.5 hours. It was cooled down to 0 °C, and then the solid compound was filtered off and washed with 5 ml ethanol. Resulted 0.015 g 2-(4-(4-(hydroxy(pyridin-4-yl)methyl)-2,5-dioxoimidazolidin-1-yl)phenyl)acetic acid (**III-3**) as a white solid compound. The organic layer was evaporated then more 0.15 ml (1.59 mmol, d = 1.137) pyridine-4-carboxaldehyde (**II**), 0.21 ml (1.5 mmol, d= 0.726) triethylamine were added and solved in 20 ml ethanol and heated and stirred at reflux temperature for 8 hours, then evaporated. It was solved in 15 ml dichloromethane and extracted with 15 ml water, and then the aqueous layer was washed with 15 ml dichloromethane. The combined organic layers were dried over magnesium sulfate and evaporated. The aqueous layer was evaporated. It was washed with 2x10 ml diisopropyl ether.

The combined solid compounds resulted 0.543 g 2-(4-(4-(hydroxy(pyridin-4-yl)methyl)-2,5-dioxoimidazolidin-1-yl)phenyl)acetic acid (**III-3**) as mixture of isomers in 1:1 ratio with 43.6% overall purity. It was solved in 10 ml glacial acetic acid and heated and stirred at 120 °C for 4 days, then evaporated. It was washed with 10 ml water, then 2 ml ethanol and 2 ml ethyl acetate and filtered off. Resulted 0.0816 g (*Z*)-2-(4-(2,5-dioxo-4-(pyridin-4-ylmethylene)imidazolidin-1-yl)phenyl)acetic acid (**I-3**) as a yellow powder.

### Example IV:

Synthesis of (*Z*)-3-((1-hydroxycyclopentyl)methyl)-5-(pyridin-4-ylmethylene)imidazolidine-2,4-dione

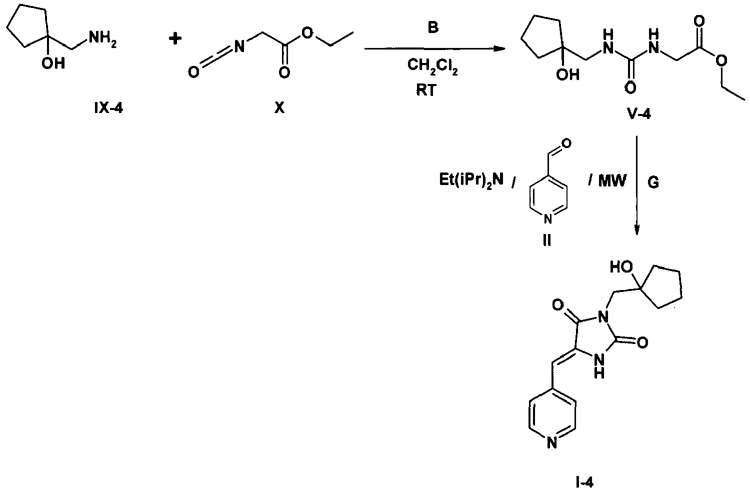

Route B, G: In a 100 mL round-bottomed flask 1 g 1-(aminomethyl)cyclopentanol (1 g, 8.68 mmol) (**IX-4**) solved 15 ml dichloromethane, then ethyl isocyanatoacetate (991.19 µl, 8.68 mmol) **(X)** was added to give a yellow solution. After 1.5 hours it was evaporated. 5 ml N-ethyl-N-isopropylpropan-2-amine and isonicotinaldehyde (817.93 µl, 8.68 mmol) was added and reacted in CEM microwave system. Conditions: 5 min ramp time, hold time: 60 min, Temperature: 165°C, Pressure: 19 bar, max power: 300W, cooling off, stirring on, Solvent: Z-empty. More isonicotinaldehyde (817.93 µl, 8.68 mmol) was added and reacted in CEM microwave under the same conditions. Purified by Combiflash Companion. Conditions: Eluent 0-3 min DCM, 3-14 min DCM to DCM:MeOH = 95:5, 14 min - DCM to DCM:MeOH = 95:5. 40 g Redisep column. It was purified again on 24 g Redisep column with the same eluent. It was washed with acetone and filtered.

Resulted 78 mg (*Z*)-3-((1-hydroxycyclopentyl)methyl)-5-(pyridin-4-ylmethylene)imidazolidine-2,4-dione (**I-4**).

### Example V:

Synthesis of (Z)-5-(pyridin-4-ylmethylene)-3-(2,2,2-trifluoroethyl)imidazolidine-2,4-dione

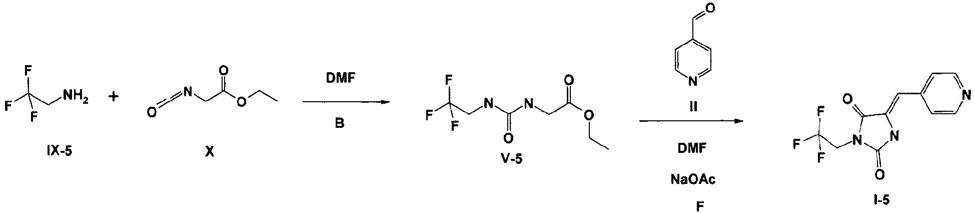

Route B, F as a 'one pot synthesis': In a 100 mL round-bottomed flask 443.97 µl (5.05 mmol, d = 1.126) 2,2,2-trifluoroethanamine (**IX-5**) solved 15 ml N,N-dimethylformamide, then 566 µl (5.05 mmol, d = 1.151) ethyl isocyanatoacetate **(X)** was added to give a colourless solution. It was stirred at room temperature for 90 minutes, then 836.53 mg (10.10 mmol) sodium acetate and 443.97 ul (10.10 mmol, d = 1.138) pyridine-4-carboxaldehyde **(II)** were added and heated up to 180 °C. It was stirred for 1 hour then evaporated, resulted a brown oil. 30 ml water was added and extracted with 3x30 ml dichloromethane. The combined organic layers were dried over sodium sulfate, and then evaporated. It was purified by Combiflash Companion. Eluent: 0-3 min DCM, 3-14 min DCM to DCM:MeOH = 95:5, 14 min - DCM to DCM:MeOH = 95:5 on 24 g Redisep column. The solid compound was washed with diisopropyl ether and filtered off. Resulted 0.404 g (Z)-5-(pyridin-4-ylmethylene)-3-(2,2,2-trifluoroethyl)imidazolidine-2,4-dione (**I-5**) with 30% yield as a yellow solid compound.

### Example VI:

Synthesis of 3-(4-methanesulfinyl-phenyl)-5-[1-pyridin-4-yl-meth-(Z)-ylidene]-imidazolidine-2,4-dione 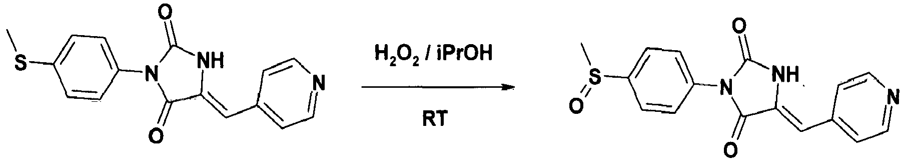 0.311 g (0.998 mmol) 3-(4-Methylsulfanyl-phenyl)-5-[1-pyridin-4-yl-meth-(Z)-ylidene]-imidazolidine-2,4-dione was solved in 30 ml 2-propanol, then 11.1 g (97.9 mmol, d = 1.11; V = 10 ml) hydrogen peroxide was added to give pale yellow suspension. It was stirred at room temperature for 12 days, and then 100 ml water was added and stirred for 1 more hour. It was left at room temperature for one more hour, then filtered off and washed with water (3x25 ml). It was dried at 100°C for 4.5 hours.
177.8 mg (yield = 54%) 3-(4-Methanesulfinyl-phenyl)-5-[1-pyridin-4-yl-meth-(Z)-ylidene]-imidazolidine-2,4-dione isolated as a light yellow powder.

### Example VII:

Synthesis of 3-(4-methanesulfonyl-phenyl)-5-[1-pyridin-4-yl-meth-(Z)-ylidene]-imidazolidine-2,4-dione 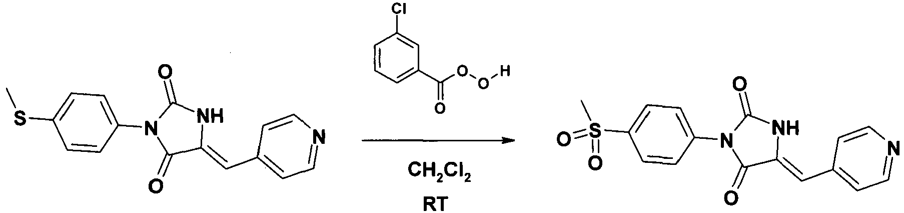 0.622 mg (2 mmol) 3-(4-Methylsulfanyl-phenyl)-5-[1-pyridin-4-yl-meth-(Z)-ylidene]-imidazolidine-2,4-dione was solved in 100 ml dichloromethane and 1.232 g (70% Aldrich, active ingredient: 0,862 g, ∼5.0 mmol) *meta*-chloroperoxybenzoic acid (mCPBA) was added. It was stirred at room temperature for 4 hours, then 50 ml saturated sodium hydrogen carbonate was added and stirred for one more hour, and then 25 ml water was added. The solid compound was filtered off and washed with water (2x10 ml), then with diisopropyl ether (2x10 ml) and dichloromethane (2x10 ml). Then it was shacked in 25 ml dichloromethane in ultrasound bath for 30 second then filtered off. Then it was stirred in 5 ml ethanol and filtered off and washed with 1 ml ethanol and diisopropylether (2x5 ml). It was recrystallized from 2.5 ml N,N-dimethylformamide, filtered off and washed with diisopropyl ether (2x1.5 ml). 101.9 mg 3-(4-Methanesulfonyl-phenyl)-5-[1-pyridin-4-yl-meth-(Z)-ylidene]-imidazolidine-2,4-dione was isolated as light yellow powder with 14.8% yield.

### Example VIII:

Synthesis of 3-{2,5-dioxo-4-[1-pyridin-4-yl-meth-(*Z*)-ylidene]-imidazolidin-1-yl}-benzoic acid 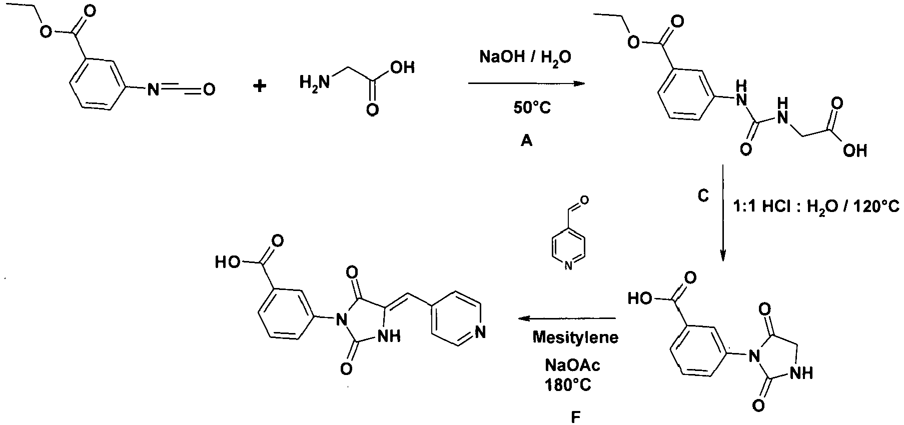 3-{2,5-Dioxo-4-[1-pyridin-4-yl-meth-(*Z*)-ylidene]-imidazolidin-1-yl}-benzoic acid synthesized from 3-isocyanato-benzoic acid ethyl ester *via* "route A" to give 3-(3-Carboxymethyl-ureido)-benzoic acid ethyl ester. 3-(3-carboxymethyl-ureido)-benzoic acid ethyl ester was cyclised in HCl *via* "route C" to give 3-(2,5-Dioxo-imidazolidin-1-yl)-benzoic acid, which was reacted *via* "route F" to give 3-{2,5-Dioxo-4-[1-pyridin-4-yl-meth-(*Z*)-ylidene]-imidazolidin-1-yl}-benzoic acid.

General method for the reaction of 3- or 4-{2,5-Dioxo-4-[1-pyridin-4-yl-meth-(*Z*)-ylidene]-imidazolidin-1-yl}-benzoic acids with different amines ("route I"): 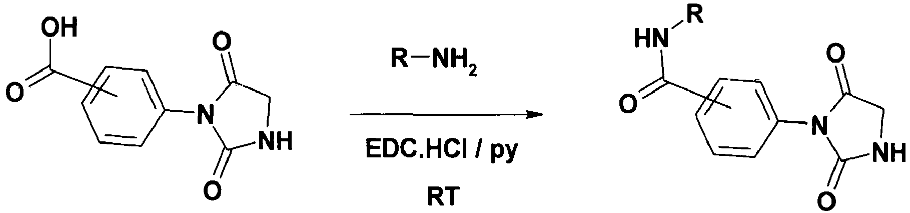 2 mmol of 3- or 4-(2,5-Dioxo-imidazolidin-1-yl)-benzoic acid was solved in 10 ml pyridine and the appropriate amine (3 mmol, 1.5 eq.) was added and 1-Ethyl-3-(3-dimethyllaminopropyl)carbodiimide hydrochloride (EDC.HCl, 3 mmol, 1.5 eq.) and it was stirred at room temperature till the disappearance of the starting material (carboxylic acid). Then it was evaporated, 50 ml water was added, washed with dichloromethane (3x50 ml). The organic layer was dried over sodium sulfate, evaporated. Purified by Combiflash Companion.

### Example IX:

Synthesis of 3-[3-(morpholine-4-carbonyl)-phenyl]-5-[1-pyridin-4-yl-meth-(Z)-ylidene]-imidazolidine-2,4-dione 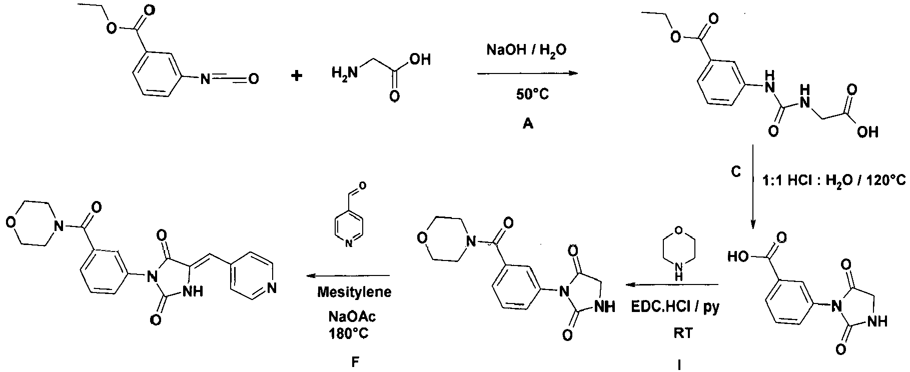 3-Isocyanato-benzoic acid ethyl ester was reacted *via* "route A" to give 3-(3-carboxymethyl-ureido)-benzoic acid ethyl ester. 3-(3-Carboxymethyl-ureido)-benzoic acid ethyl ester was cyclised in HCl *via* "route C" to give 3-(2,5-Dioxo-imidazolidin-1-yl)-benzoic acid. 3-(2,5-Dioxo-imidazolidin-1-yl)-benzoic acid was reacted via "route I" and "route F" to give 3-[3-(Morpholine-4-carbonyl)-phenyl]-5-[1-pyridin-4-yl-meth-(Z)-ylidene]-imidazolidine-2,4-dione.

### Example X:

Synthesis of 3-[4-(morpholine-4-carbonyl)-phenyl]-5-[1-pyridin-4-yl-meth-(*Z*)-ylidene]-imidazolidine-2,4-dione 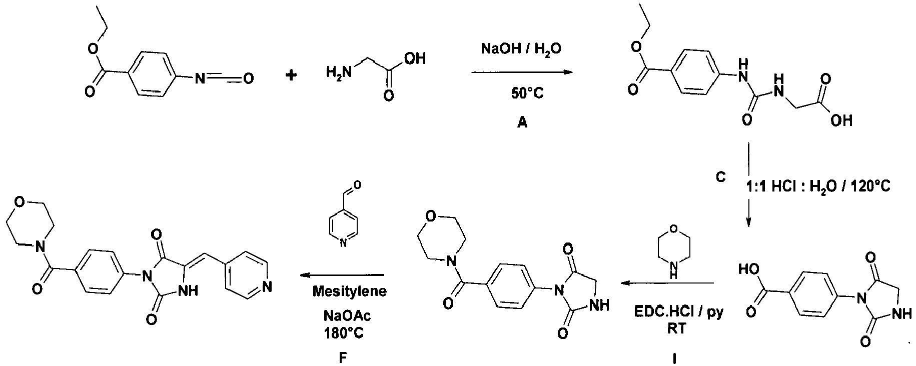 4-Isocyanato-benzoic acid ethyl ester was reacted *via* "route A" to give 4-(3-carboxymethyl-ureido)-benzoic acid ethyl ester. 4-(3-carboxymethyl-ureido)-benzoic acid ethyl ester was cyclised in HCl *via* "route C" to give 4-(2,5-Dioxo-imidazolidin-1-yl)-benzoic acid. 4-(2,5-Dioxo-imidazolidin-1-yl)-benzoic acid was reacted *via* "route I" and "route F" to give 3-[4-(Morpholine-4-carbonyl)-phenyl]-5-[1-pyridin-4-yl-meth-(*Z*)-ylidene]-imidazolidine-2,4-dione.

### Example XI:

Synthesis of 3-{2,5-dioxo-4-[1-pyridin-4-yl-meth-(*Z*)-ylidene]-imidazolidin-l-yl}-N-(2-morpholin-4-yl-ethyl)-benzamide 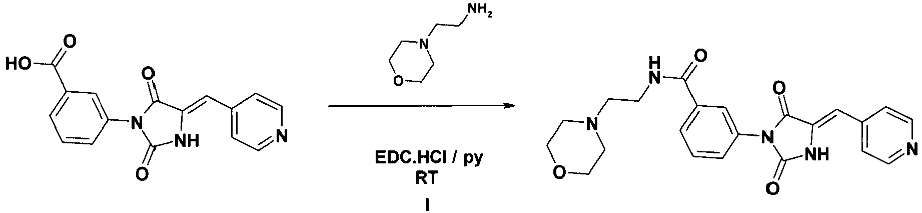 3-{2,5-Dioxo-4-[1-pyridin-4-yl-meth-(Z)-ylidene]-imidazolidin-1-yl}-N-(2-morpholin-4-yl-ethyl)-benzamide was synthesized from 3-{2,5-Dioxo-4-[1-pyridin-4-yl-meth-(*Z*)-ylidene]-imidazolidin-1-yl}-benzoic acid *via* "route I".

### Example XII:

Synthesis of {2,5-dioxo-4-[1-pyridin-4-yl-meth-(*Z*)-ylidene]-imidazolidin-1-yl}-acetic acid ethyl ester 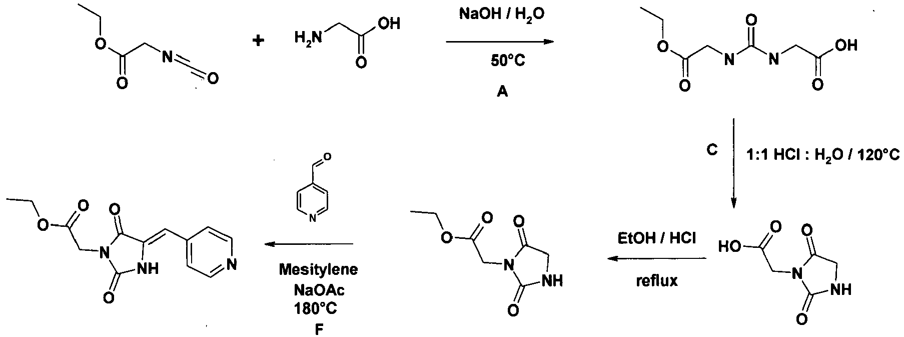 (3-Ethoxycarbonylmethyl-ureido)-acetic acid was synthesized *via* "route A" from isocyanato-acetic acid ethyl ester. (3-Ethoxycarbonylmethyl-ureido)-acetic acid was cyclised *via* "route C" in HCl to give (2,5-Dioxo-imidazolidin-1-yl)-acetic acid.

Synthesis of (2,5-Dioxo-imidazolidin-1-yl)-acetic acid ethyl ester
0.531 g (3.358 mmol) (2,5-Dioxo-imidazolidin-1-yl)-acetic acid was solved in 10 ml ethanol and 0.1 ml cc. HCl was added. It was heated at reflux temperature for 5.5 hours, and then evaporated. 0.608 g (2,5-Dioxo-imidazolidin-1-yl)-acetic acid ethyl ester was isolated with 97.25% yield.
{2,5-Dioxo-4-[1-pyridin-4-yl-meth-(Z)-ylidene]-imidazolidin-1-yl}-acetic acid ethyl ester was synthesized from (2,5-Dioxo-imidazolidin-1-yl)-acetic acid ethyl ester *via* "route F".

### Example XIII:

Synthesis of 3-[4-((2*R*,6*S*)-2,6-dimethyl-morpholine-4-carbonyl)-phenyl]-5-[1-pyridin-4-yl-meth-(Z)-ylidene]-imidazolidine-2,4-dione 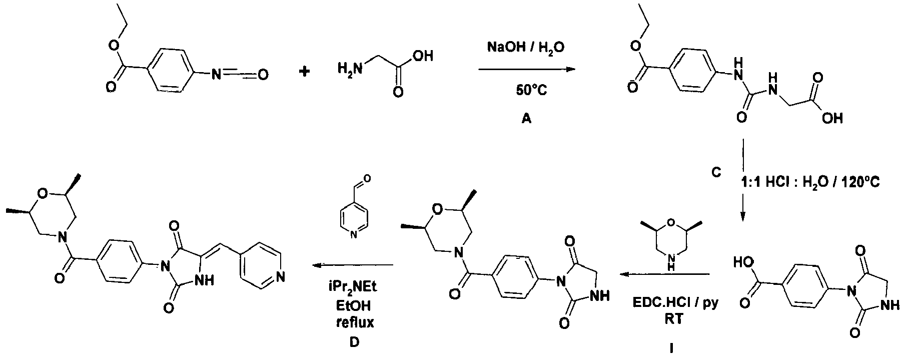 4-Isocyanato-benzoic acid ethyl ester was reacted *via* "route A" to give 4-(3-carboxymethyl-ureido)-benzoic acid ethyl ester. 4-(3-carboxymethyl-ureido)-benzoic acid ethyl ester was cyclised in HCl *via* "route C" to give 4-(2,5-Dioxo-imidazolidin-1-yl)-benzoic acid. 4-(2,5-Dioxo-imidazolidin-1-yl)-benzoic acid was reacted *via* "route I" to give 3-[4-((2*R*,6*S*)-2,6-Dimethyl-morpholine-4-carbonyl)-phenyl]-imidazolidine-2,4-dione. 3-[4-((2*R*,6*S*)-2,6-Dimethyl-morpholine-4-carbonyl)-phenyl]-imidazolidine-2,4-dione was reacted with pyridine-4-carboxaldehyde *via* "route D" to give 3-[4-((2*R*,6*S*)-2,6-Dimethyl-morpholine-4-carbonyl)-phenyl]-5-[1-pyridin-4-yl-meth-(Z)-ylidene]-imidazolidme-2,4-dione.

### Example XIV:

Synthesis of {2,5-dioxo-4-[1-pyridin-4-yl-meth-(*Z*)-ylidene]-imidazolidin-1-yl}-acetic acid 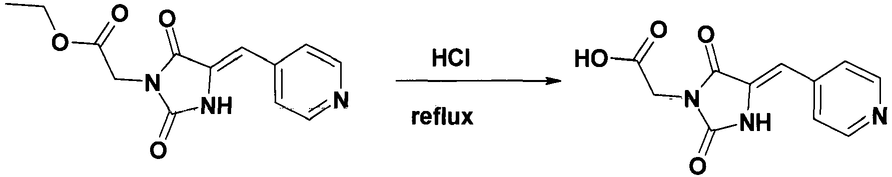 0.37 g (1,34 mmol) {2,5-Dioxo-4-[1-pyridin-4-yl-meth-(Z)-ylidene]-imidazolidin-1-yl}-acetic acid ethyl ester was solved in 1:1 HCl in water and heated and stirred at reflux temperature for 3 hours. The solid compound was filtered off and washed with water (2x5 ml). The mother liquor was evaporated and resulted in a solid compound which was filtered off and washed with ethanol (2x5 ml). The combined solid compounds were mixed and washed with ethanol (5 ml, then 2x2 ml).
0.216 g {2,5-Dioxo-4-[1-pyridin-4-yl-meth-(Z)-ylidene]-imidazolidin-1-yl}-acetic acid isolated with 65% yield as a light green powder.

### Example XV:

Synthesis of 3-[4-(4-methyl-piperidine-1-carbonyl)-phenyl]-5-[1-pyridin-4-yl-meth-(Z)-ylidene]-imidazolidine-2,4-dione 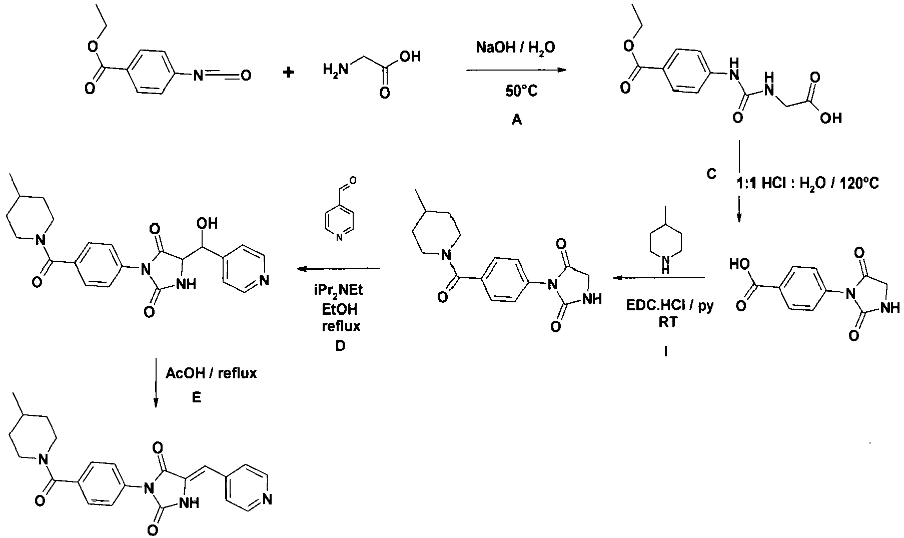 4-Isocyanato-benzoic acid ethyl ester was reacted *via* "route A" to give 4-(3-carboxymethyl-ureido)-benzoic acid ethyl ester. 4-(3-carboxymethyl-ureido)-benzoic acid ethyl ester was cyclised in HCl *via* "route C" to give 4-(2,5-Dioxo-imidazolidin-1-yl)-benzoic acid. 4-(2,5-Dioxo-imidazolidin-1-yl)-benzoic acid was reacted *via* "route I" to give 3-[4-(4-Methylpiperidine-1-carbonyl)-phenyl]-imidazolidine-2,4-dione. 3-[4-(4-Methyl-piperidine-1-carbonyl)-phenyl]-5-[1-pyridin-4-yl-meth-(Z)-ylidene]-imidazolidine-2,4-dione was synthesized from 3-[4-(4-Methyl-piperidine-1-carbonyl)-phenyl]-imidazolidine-2,4-dione *via* "route D" and "route E".

### Example XVI:

Synthesis of 5-[1-pyridin-4-yl-meth-(Z)-ylidene]-3-[4-(pyrrolidine-1-carbonyl)-phenyl]-imidazolidine-2,4-dione 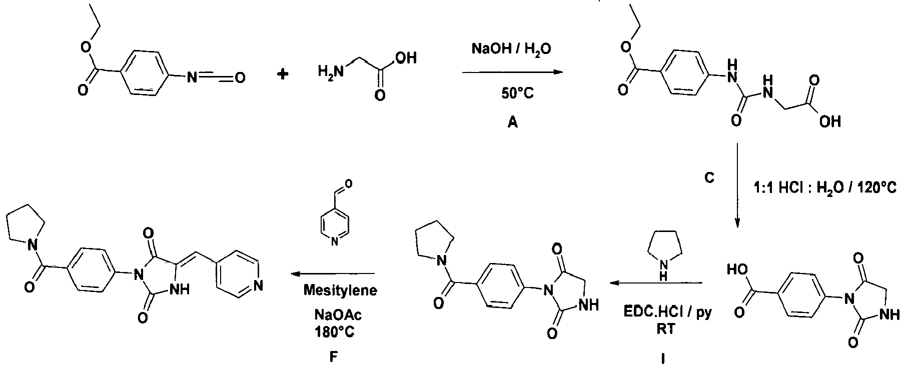 4-Isocyanato-benzoic acid ethyl ester was reacted *via* "route A" to give 4-(3-carboxymethyl-ureido)-benzoic acid ethyl ester. 4-(3-carboxymethyl-ureido)-benzoic acid ethyl ester was cyclised in HCl *via* "route C" to give 4-(2,5-Dioxo-imidazolidin-1-yl)-benzoic acid. 5-[1-Pyridin-4-yl-meth-(Z)-ylidene]-3-[4-(pyrrolidine-1-carbonyl)-phenyl]-imidazolidine-2,4-dione was synthesized from 4-(2,5-Dioxo-imidazolidin-1-yl)-benzoic acid *via* "route I" and "route F".

### Example XVII:

Synthesis of N-cyclopentyl-4-{2,5-dioxo-4-[1-pyridin-4-yl-meth-(Z)-ylidene]-imidazolidin-1-yl}-benzamide

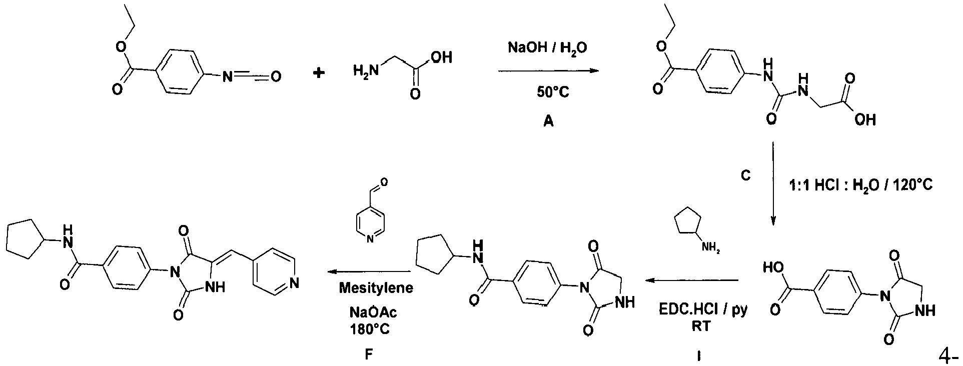

Isocyanato-benzoic acid ethyl ester was reacted *via* "route A" to give 4-(3-carboxymethyl-ureido)-benzoic acid ethyl ester. 4-(3-carboxymethyl-ureido)-benzoic acid ethyl ester was cyclised in HCl *via* "route C" to give 4-(2,5-Dioxo-imidazolidin-1-yl)-benzoic acid. N-Cyclopentyl-4-{2,5-dioxo-4-[1-pyridin-4-yl-meth-(Z)-ylidene]-imidazolidin-1-yl}-benzamide was synthesized from 4-(2,5-Dioxo-imidazolidin-1-yl)-benzoic acid *via* "route I" and "route F".

### Example XVIII:

Synthesis of N-cyclopropyl-4-{2,5-dioxo-4-[1-pyridin-4-yl-meth-(Z)-ylidene]-imidazolidin-1-yl}-benzamide 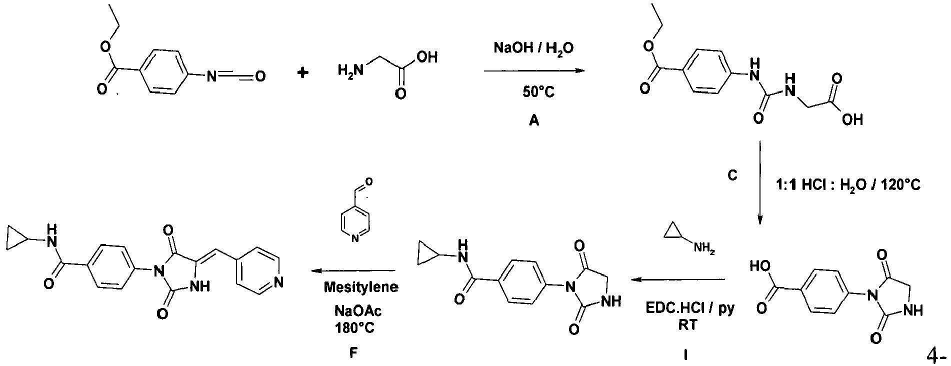 Isocyanato-benzoic acid ethyl ester was reacted *via* "route A" to give 4-(3-carboxymethyl-ureido)-benzoic acid ethyl ester. 4-(3-carboxymethyl-ureido)-benzoic acid ethyl ester was cyclised in HCl *via* "route C" to give 4-(2,5-Dioxo-imidazolidin-1-yl)-benzoic acid. N-Cyclopropyl-4-{2,5-dioxo-4-[1-pyridin-4-yl-meth-(Z)-ylidene]-imidazolidin-1-yl}-benzamide was synthesized from 4-(2,5-Dioxo-imidazolidin-1-yl)-benzoic acid *via* "route I" and "route F".

### Example XIX:

Synthesis of N-cyclohexyl-4-{2,5-dioxo-4-[1-pyridin-4-yl-meth-(Z)-ylidene]-imidazolidin-1-yl}-benzamide 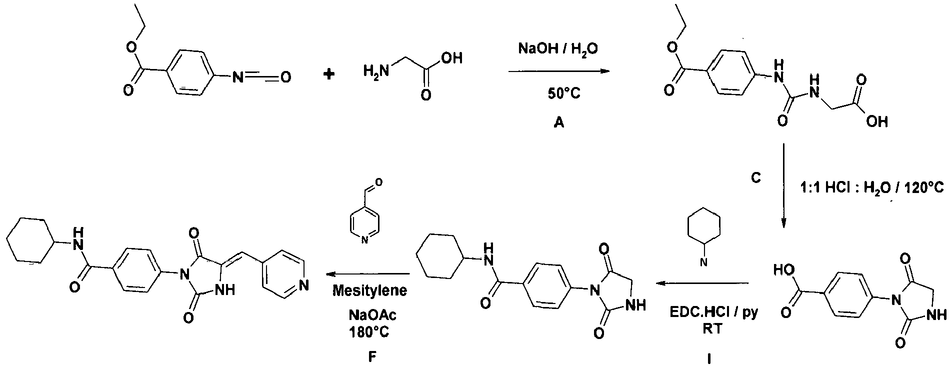 4-Isocyanato-benzoic acid ethyl ester was reacted *via* "route A" to give 4-(3-carboxymethyl-ureido)-benzoic acid ethyl ester. 4-(3-carboxymethyl-ureido)-benzoic acid ethyl ester was cyclised in HCl *via* "route C" to give 4-(2,5-Dioxo-imidazolidin-1-yl)-benzoic acid. N-Cyclohexyl-4-{2,5-dioxo-4-[1-pyridin-4-yl-meth-(Z)-ylidene]-imidazolidin-1-yl}-benzamide was synthesized from 4-(2,5-Dioxo-imidazolidin-1-yl)-benzoic acid *via* "route I" and "route F".

### Example XX:

Synthesis of 1-(4-{2,5-dioxo-4-[1-pyridin-4-yl-meth-(Z)-ylidene]-imidazolidin-1-yl}-benzoyl)-piperidine-4-carboxylic acid ethyl ester 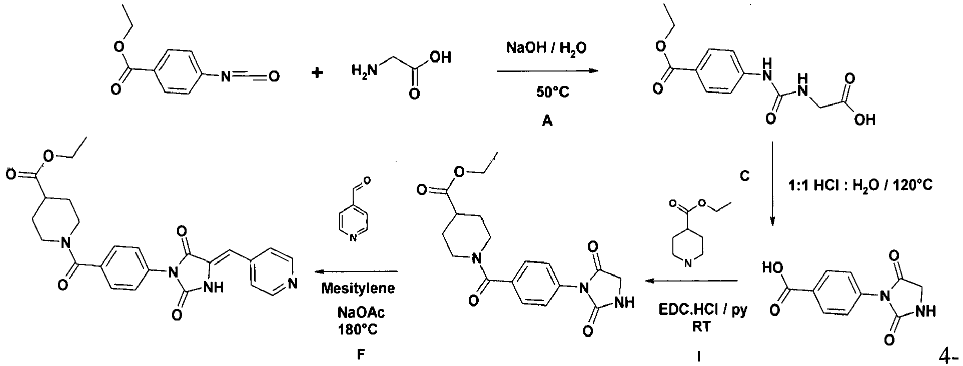 Isocyanato-benzoic acid ethyl ester was reacted *via* "route A" to give 4-(3-carboxymethyl-ureido)-benzoic acid ethyl ester. 4-(3-carboxymethyl-ureido)-benzoic acid ethyl ester was cyclised in HCl *via* "route C" to give 4-(2,5-Dioxo-imidazolidin-1-yl)-benzoic acid. 1-(4-{2,5-Dioxo-4-[1-pyridin-4-yl-meth-(Z)-ylidene]-imidazolidin-1-yl}-benzoyl)-piperidine-4-carboxylic acid ethyl ester was synthesized from 4-(2,5-Dioxo-imidazolidin-1-yl)-benzoic acid *via* "route I" and "route F".

The compounds of formula I prepared according to the present invention are presented in Table 1.

| **Comp ound No.** | **Chemistry** | **LCMS (M+H)** | **Purity (%)** | **Rt (min) TFA** | **Rt (min) NH₄OAc** | **Method of synthesis** |
|---|---|---|---|---|---|---|
| 1 | 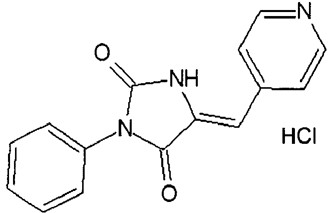 | 266 | 96.2 | 1.92 | | A, C, F, HCl in diethyl ether |
| 2 | 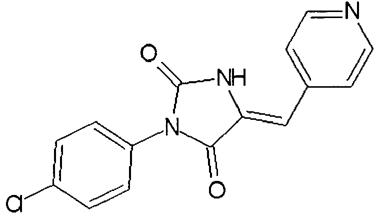 | 300 | 99.6 | 2.28 | | A,C,F F |
| 3 | 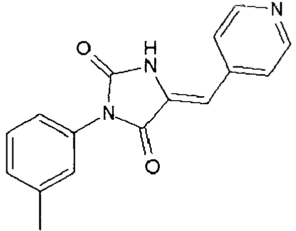 | 280 | 97.3 | 2.19 | | A,C,F F |
| 4 | 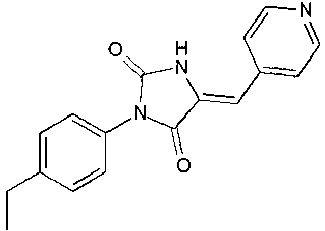 | 294 | 99.3 | 2.50 | | A, C, F |
| 5 | 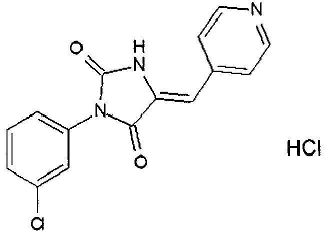 | 300 | 97.1 | 2.30 | | A, C, F |
| 6 | 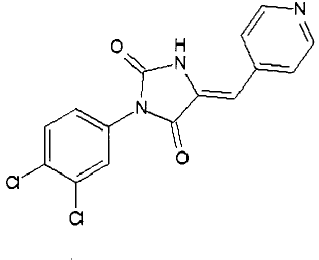 | 334 | 97.4 | | 5.15 | A, C, F |
| 7 | 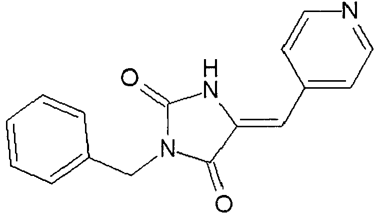 | 280 | 94.6 | 2.25 | | A, C, F |
| 8 | 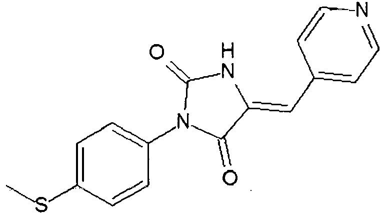 | 312 | 95.8 | 2.38 | | A, C, F |
| 9 | 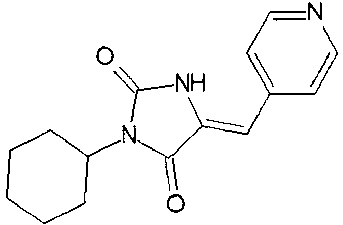 | 272 | 94.2 | 2.20 | | A, C, H |
| 10 | 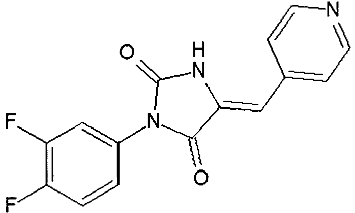 | 302 | 97.7 | | 4.40 | A, C, F, Example I compound I-1 |
| 11 | 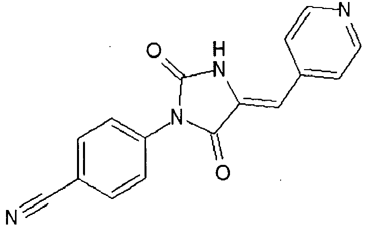 | 291 | 94.2 | | 3.88 | A, C, F |
| 12 | 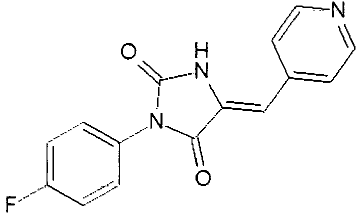 | 284 | 96.7 | | 4.06 | A, C, F |
| 13 | 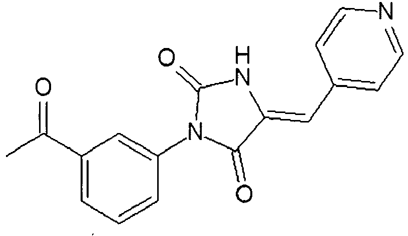 | 308 | 97.9 | 1.82 | | A, C, F |
| 14 | 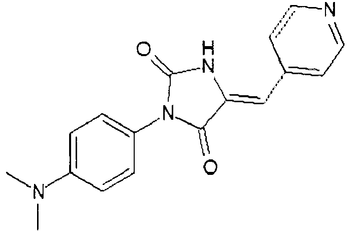 | 309 | 96.1 | | 4.32 | A, C, F |
| 15 | 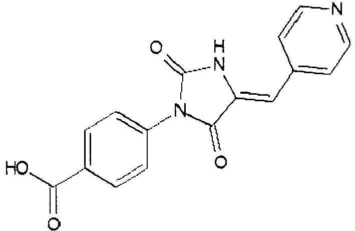 | 310 | 93.7 | 1.54 | | A, C, F |
| 16 | 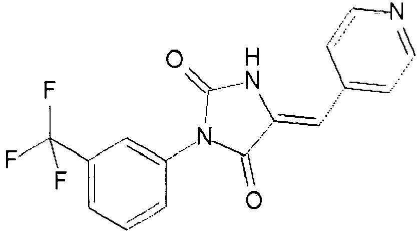 | 334 | 97.7 | 2.38 | | A, C, F |
| 17 | 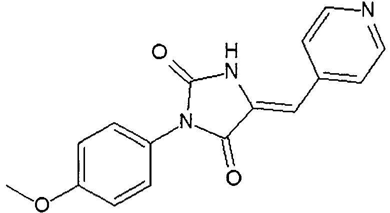 | 296 | 98.5 | 1.91 | | A, C, F |
| 18 | 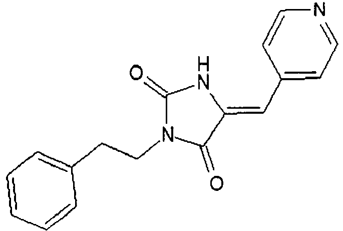 | 294 | 87.6 | 4.49 | | A, C, F |
| 19 | 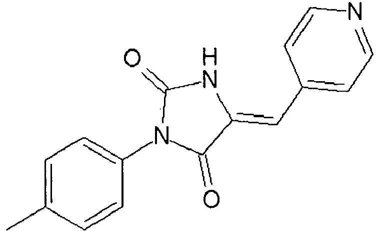 | 280 | 97.4 | 4.34 | | A, C, F |
| 20 | 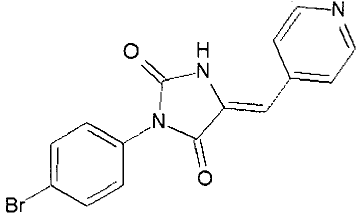 | 345 | 99.8 | | 4.73 | A, C, F |
| 21 | 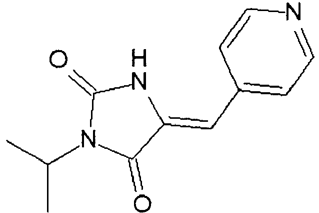 | 232 | 95.5 | 1.74 | | A, C, F |
| 22 | 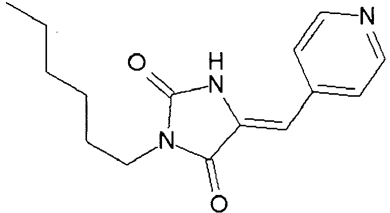 | 274 | 91.7 | 2.50 | | A, C, F |
| 23 | 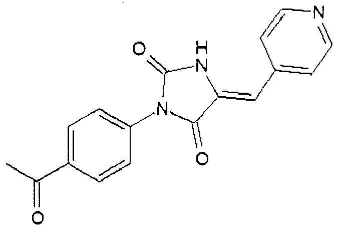 | 308 | 96.5 | 2.04 | | A, C, F |
| 24 | 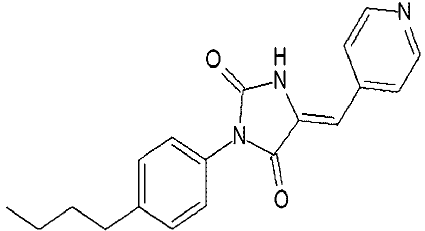 | 322 | 95.6 | 2.82 | | A, C, F |
| 25 | 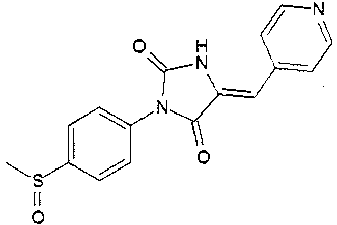 | 328 | 94.8 | 1.56 | | Example VI. |
| 26 | 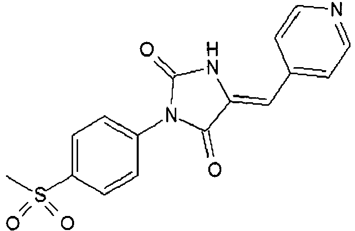 | 344 | 91.8 | 2.23 | | Example VII. |
| 27 | 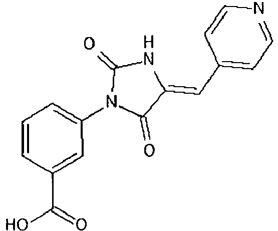 | 310 | 97.5 | 1.77 | | Example VIII. |
| 28 | 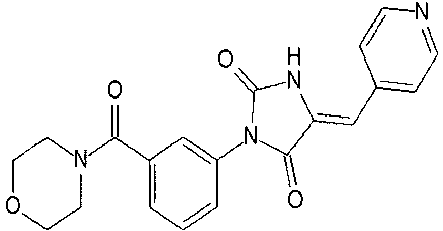 | 379 | 95.0 | 1.76 | | Example IX. |
| 29 | 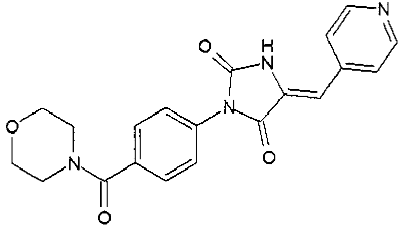 | 379 | 98.6 | 1.56 | | Example X. |
| 30 | 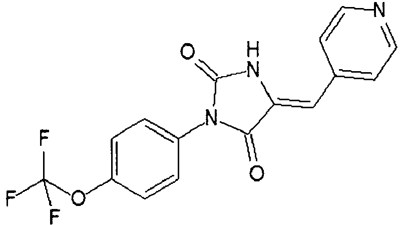 | 350 | 93.2 | 2.54 | | A, C, H Example II. Compound I-2 |
| 31 | 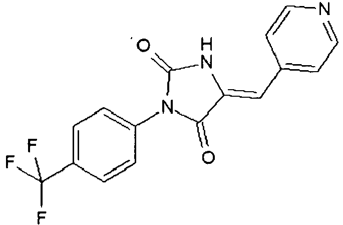 | 334 | 96.9 | 2.39 | | A, C, D, E |
| 32 | 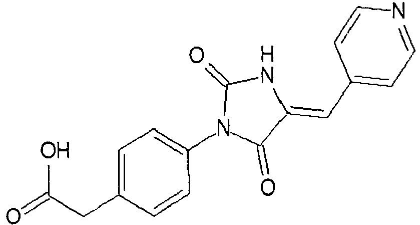 | 324 | 90.2 | 1.60 | | A, C, D, E Example III. Compound I-3 |
| 33 | 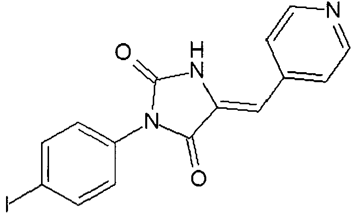 | 391 | 92.5 | 2.35 | | A, C, D, E |
| 34 | 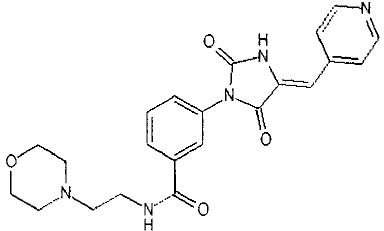 | 422 | 93.2 | 1.37 | | Example XI. |
| 35 | 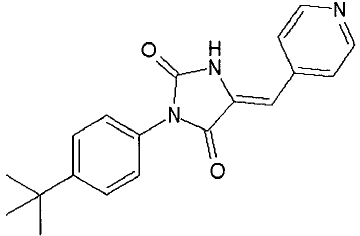 | 322 | 97.2 | 2.66 | | A, C, D, E |
| 36 | 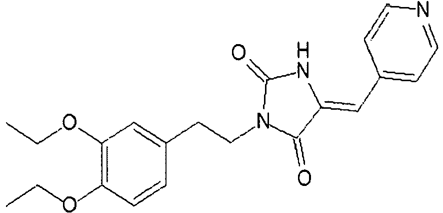 | 382 | 98.1 | 2.31 | | B, C, H |
| 37 | 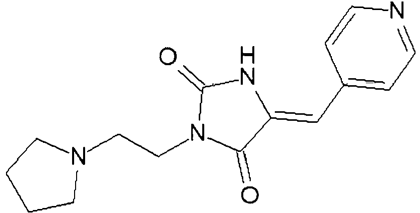 | 287 | 96.5 | | 2.19 | B, C, D, E |
| 38 | 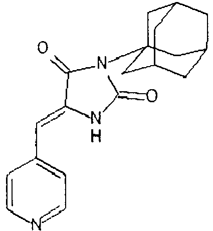 | 324 | 98.6 | 2.68 | | B, C, D, E |
| 39 | 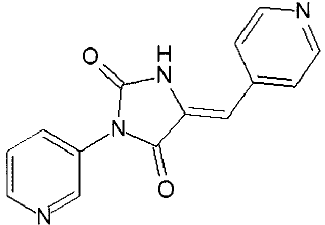 | 267 | 98.1 | | 2.84 | B, C, D, E |
| 40 | 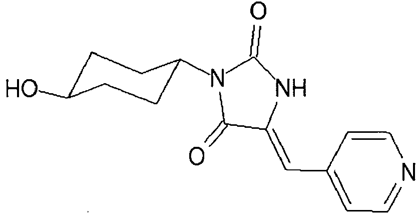 | 288 | 95.1 | 1.32 | | B, C, G |
| 41 | 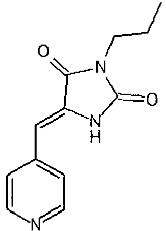 | 232 | 96.8 | 1.65 | | B, C, G |
| 42 | 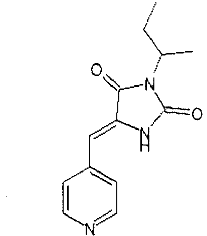 | 246 | 99.4 | 1.89 | | B, C, G |
| 43 | 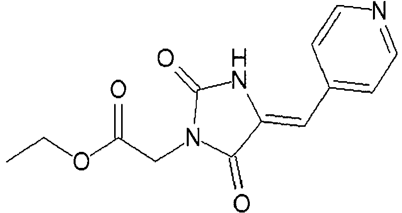 | 276 | 97.2 | 1.60 | | Example XII. |
| 44 | 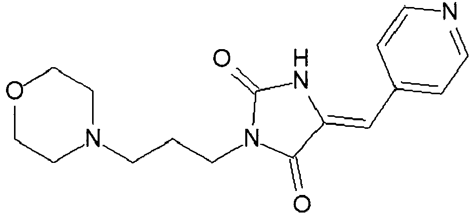 | 317 | 98.6 | 0.59 | | B, C, F |
| 45 | 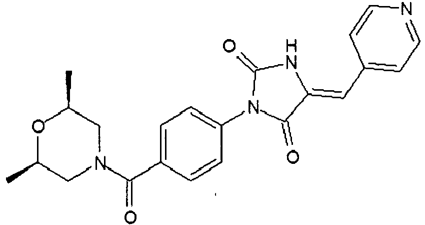 | 407 | 96.6 | 1.84 | | Example XIII. |
| 46 | 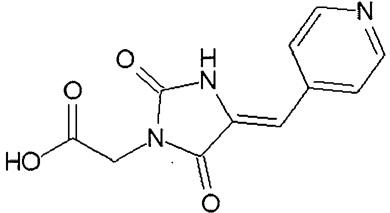 | 248 | 99.0 | 0.78 | | Example XIV. |
| 47 | 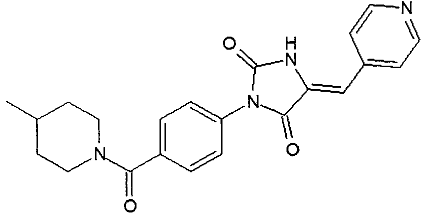 | 391 | 93.2 | 2.17 | | Example XV. |
| 48 | 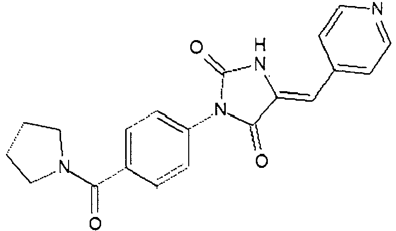 | 363 | 93.6 | 1.74 | | Example XVI. |
| 49 | 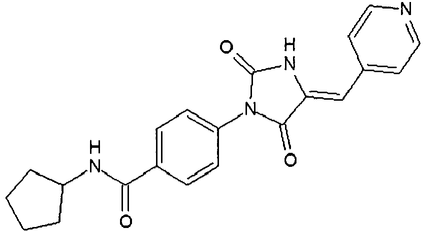 | 377 | 95.4 | 2.06 | | Example XVII. |
| 50 | 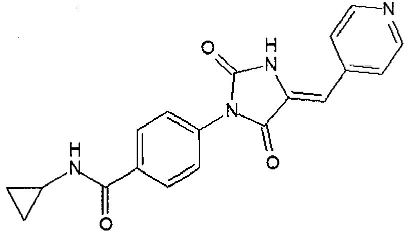 | 349 | 96.7 | 1.62 | | Example XVIII |
| 51 | 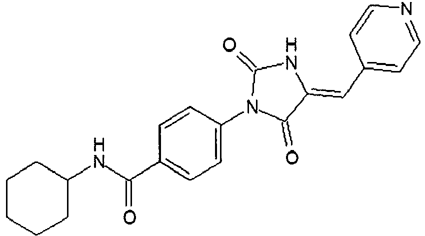 | 391 | 94.2 | 2.25 | | Example XIX. |
| 52 | 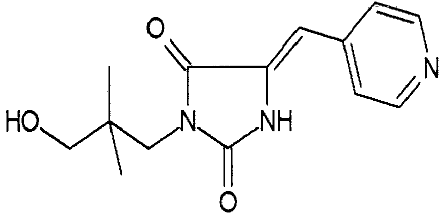 | 276 | 92.7 | 1.52 | | B, C, G |
| 53 | 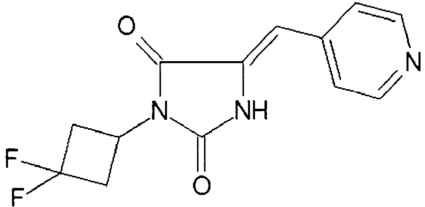 | 280 | 93.9 | 1.84 | | B, G |
| 54 | 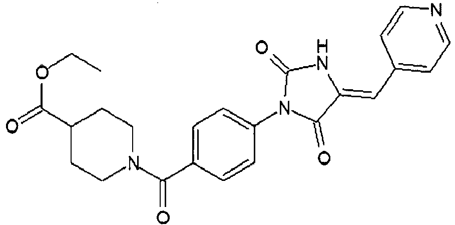 | 449 | 98.3 | 2.04 | | Example XX. |
| 55 | 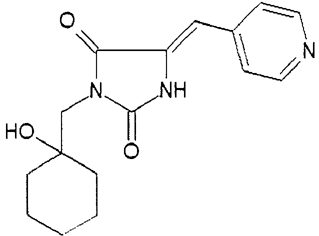 | 302 | 96.3 | 1.79 | | B, G |
| 56 | 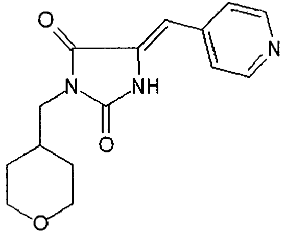 | 288 | 97.0 | 1.54 | | B, C, G |
| 57 | 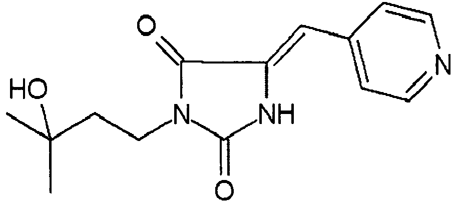 | 276 | 98.1 | 1.46 | | B, C, G |
| 58 | 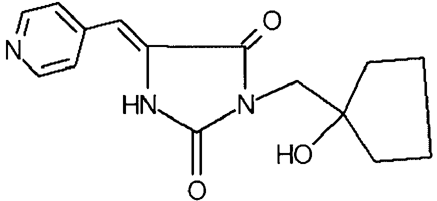 | 288 | 97.6 | 1.60 | | B, G Example IV. Compound I-4 |
| 59 | 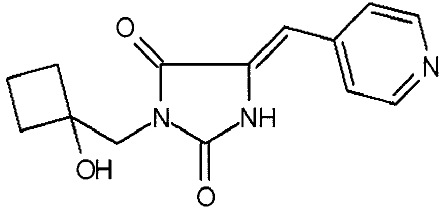 | 274 | 97.7 | 1.43 | | B, C, F |
| 60 | 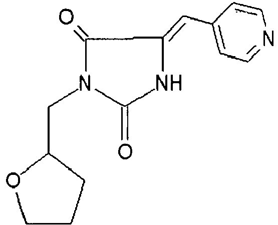 | 274 | 99.6 | 1.51 | | B, C, G |
| 61 | 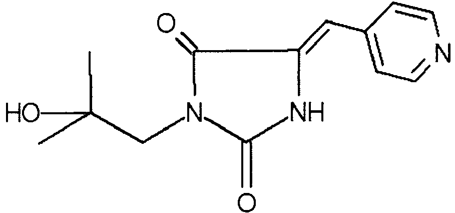 | 262 | 96.2 | 1.27 | | B, C, F |
| 62 | 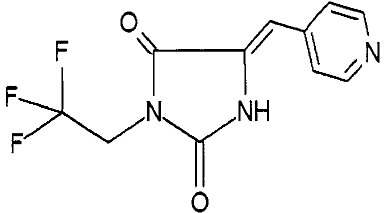 | 272 | 96.8 | 1.65 | | B, F, Example V. Compound I-5 |
| 63 | 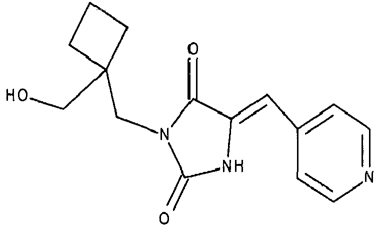 | 288 | 94.3 | 2.76 | | B, F |
| 64 | 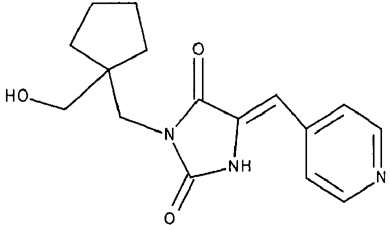 | 302 | 95.6 | 1.80 | | B, F |
| 65 | 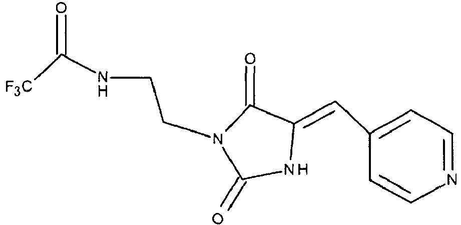 | 329 | 98.1 | 1.43 | | B, F |

### Biological methods:

Enzyme assay on human recombinant CD38 enzyme were performed in order to determine the in vitro potency of the newly synthesized compounds. IC50 values were determined for the test compounds and used to explore the structure-activity relationships (SAR). The established SAR was used to feed back the molecular design and to suggest some suitable modifications for groups and structural elements by which the activity of test compounds for humans CD38 enzyme would be improved.

The most active compounds were also investigated on natural cell surface human CD38 enzyme. A549 human lung epithelial cells were used as enzyme source. The A549 cells express active CD38 enzyme on the cell surface. The investigated, most active inhibitors of human recombinant CD38 enzyme show high activity on the cell surface natural enzyme too.

N6-etheno-NAD (ε-NAD), a NAD analogue previously shown to be a convenient substrate of CD38 enzyme activity (Graeff et al. J. Biol. Chem. 269, pp 30260-30367, 1194) was used to measure the activity of human recombinant CD38 and cell surface enzyme activity.

### Human recombinant enzyme assay:

A fluorimetric CD38 enzyme assay was performed as described by Graeff et al. The reaction was started with 10 µl of N6-etheno-NAD (ε-NAD) substrate, final concentration 100 µM, in a final volume of 0.1 ml buffered with 50 mM HEPES, 0.05% BRIJ, pH = 6.0 containing 100 ng/ml recombinant CD38 (R&D Systems, H7006). Cleavage of ε-NAD, which causes a 10-fold fluorescence enhancement, was continuously followed at 37°C in a BMG Polarstar spectrofluorometer at an emission wavelength of 410 nm and an excitation of 300 nm, reaction time was 30 min. NADase activity was expressed as Δ relative fluorescence/min. The rate of enzyme reactions were compared in the presence and absence of inhibitor molecule, the IC50 values were calculated with Biostat Speed program.

### Cell surface enzyme assay on A549 cells:

### Culturing of A549 cells:

Type II-like human lung epithelial cells A 549 (ATCC CCL 185) were grown in RPMI 1640 medium supplemented with 10% fetal calf serum, 2mM 1-glutamine and penicillin/streptomycin 1% in a humidified incubator with an atmosphere of 95% air and 5% CO2 at 37 °C. At about 70-80% confluence, cells were detached with trypsin/EDTA solution to form a cell suspension and counted. Cell viability was higher as 95% using trypan blue dye. Cells were plated at a density of 500.000 cells/well (24 well plate), before 24 hours of starting the enzyme assay. The medium was removed and replaced by assay buffer (20 mM HEPES, pH=7.4 containing 140 mM NaCl, 5 mM KCl, 1 mM MgSO₄ 1 mM CaCl₂, 1mM Na₂HPO₄, 5.5 mM glucose).

A549 cells (800.000/well, 500 µl) were pre-incubated in the presence and absence of inhibitors for 15 min before the addition of ε-NAD, final substrate concentration was 100 µM. After 60 min, the plate was centrifuged and 100 µl of supernatant was removed and measured fluorimetrically in BMG Polarstar spectrofluorimeter at an emission wavelength of 410 nm and an excitation of 300 nm. The mean difference in the fluorescence between reaction stopped at 0 and 60 min was used an arbitrary unit of NADase activity. The NADase activity in the absence and presence of inhibitors were compared and IC50 values were calculated.

### Results:

The exemplified compounds of the present invention have activities in the human recombinant enzyme assay of less than 10 micromolar, more particular compound have activities of less than 1 micromolar, and further particular compounds have activities of less than 200 nanomolar IC50. The compounds tested in the above cell surface enzyme assay on A549 cells displayed an IC50 value of less than 1 micromolar.

The IC50 values of the compounds tested in the above assays are shown in Table 2. IC50 ENAD values correspond to the activity of the compound as measured in the human recombinant enzyme assay. IC50 A549 values correspond to the activity of the compounds as measured in the cell surface enzyme assay on A549 cells.

| **Compound No.** | **IC50 (nM) ENAD** | **IC50 (nM) A549** |
|---|---|---|
| **1** | **30** | |
| **2** | **30** | **181** |
| **3** | **50** | |
| **4** | **60** | |
| **5** | **50** | |
| **6** | **50** | |
| **7** | **50** | |
| **8** | **70** | |
| **9** | **292** | |
| **10** | **30** | |
| **11** | **30** | |
| **12** | **16** | |
| **13** | **30** | |
| **14** | **90** | |
| **15** | **110** | |
| **16** | **77** | |
| **17** | **55** | |
| **18** | **60** | |
| **19** | **30** | |
| **20** | **20** | |
| **21** | **40** | |
| **22** | **55** | |
| **23** | **26** | |
| **24** | **80** | **297** |
| **25** | **60** | |
| **26** | **22** | |
| **27** | **56** | |
| **28** | **50** | **359** |
| **29** | **51** | |
| **30** | **28** | |
| **31** | **43** | |
| **32** | **38** | |
| **33** | **38** | **272** |
| **34** | **65** | |
| **35** | **100** | |
| **36** | **250** | |
| **37** | **290** | |
| **38** | **400** | |
| **39** | **61** | |
| **40** | **214** | |
| **41** | **80** | |
| **42** | **868** | |
| **43** | **224** | |
| **44** | **151** | |
| **45** | **34** | |
| **46** | **490** | |
| **47** | **74** | |
| **48** | **52** | |
| **49** | **50** | |
| **50** | **63** | |
| **51** | **63** | |
| **52** | **42** | |
| **53** | **50** | **249** |
| **54** | **36** | |
| **55** | **55** | **242** |
| **56** | **20** | **137** |
| **57** | **20** | |
| **58** | **30** | **246** |
| **59** | **35** | **98.5** |
| **60** | **22** | **81.8** |
| **61** | **25** | **288** |
| **62** | **57** | **604** |
| **63** | **12** | |
| **64** | **16** | |
| **65** | **240** | |

### Formulation Examples

### (1) Tablets

The ingredients below are mixed by an ordinary method and compressed by using a conventional apparatus.

| | |
|---|---|
| Compound of Example I | 30 mg |
| Crystalline cellulose | 60 mg |
| Corn starch | 100 mg |
| Lactose | 200 mg |
| Magnesium stearate | 4 mg |

### (2) Soft capsules

The ingredients below are mixed by an ordinary method and filled in soft capsules.

| | |
|---|---|
| Compound of Example I | 30 mg |
| Olive oil | 300 mg |
| Lecithin | 20 mg |

### (1) Parenteral preparations

The ingredients below are mixed by an ordinary method to prepare injections contained in a 1 ml ampoule.

| | |
|---|---|
| Compound of Example I | 3 mg |
| Sodium chloride | 4 mg |
| Distilled water for injection | 1 ml |

The compounds of the present invention have CD38 inhibitory activity and are useful as an active ingredient of a medicament for preventive and/or therapeutic treatment of inflammatory diseases such as COPD, IBD asthma, allergy, obesity, metabolic syndrome, diabetic nephropathy, cardiac hypertrophy and cerebral ischemia.

## Claims

1. A compound of formula I 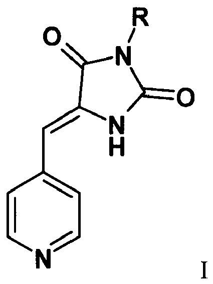 wherein
R represents a group of formula II 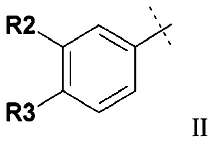 wherein
R2 and R3 represent independently from each other H, halogen, cyano, C₁-C₄ alkyl, acetyl, C₁-C₄ halogenalkyl, C₁-C₄ alkyloxy, C₁-C₄ halogenalkoxy, C₁-C₄ alkylthio, C₁-C₄ alkylsulfinyl, C₁-C₄ alkylsulfonyl, -COOH, -(C₁-C₄ alkyl)-COOH, -NR4R5, wherein R4 and R5 are independently from each other H or C₁-C₄ alkyl; or -CO-NRxRy, wherein
Rx represents C₃-C₆ cycloalkyl or C₁-C₂ alkyl substituted with morpholinyl, and Ry represents H, or
Rx and Ry form together with the nitrogen attached a 5 or 6 membered heterocyclic ring optionally containing one further heteroatom selected from O and N and optionally substituted with one or more substituents selected from the group consisting of C₁-C₂ alkyl and -COO-(C₁-C₄ alkyl); or
R represents C₁-C₆ alkyl optionally substituted with one or more substituents selected from the group consisting of -OH, -COOH, -COO-(C₁-C₄ alkyl), -NH-CO-CF₃, -CF₃, C₃-C₆ cycloalkyl, optionally substituted by -OH or -CH₂-OH; and a 5 or 6 membered heterocyclic ring optionally containing one or two heteroatom selected from O, N and S; or
R represents C₃-C₆ cycloalkyl optionally substituted with one or more substituents selected from the group consisting of OH and halogen; or
R represents phenyl-(C₁-C₂ alkyl), optionally substituted by one or more C₁-C₄ alkoxy; adamantyl, or pyridyl;
or a pharmaceutically acceptable salt or stereoisomer thereof or a pharmaceutically acceptable salt of the stereoisomer.

2. A compound of formula I according to claim 1, wherein
R represents a group of formula II
wherein R2 and R3 represent independently from each other H, halogen, cyano, C₁-C₄ alkyl, acetyl, trifluoromethyl, methoxy, trifluoromethoxy, methylthio, methylsulfinyl, methylsulfonyl, -COOH, - CH₂-COOH, dimethylamino; or -CO-NRxRy, wherein
Rx represents C₃-C₆ cycloalkyl or morpholinyl-ethyl, and Ry represents H, or
Rx and Ry form together with the nitrogen attached a pyrrolidinyl, a pyperidinyl or a morpholinyl ring optionally substituted with one or two substituents selected from the group consisting of methyl and ethoxy-carbonyl; or
R represents C₁-C₆ alkyl optionally substituted with one or more substituents selected from the group consisting of -OH, -COOH, -COO-(C₁-C₂ alkyl), -NH-CO-CF₃, -CF₃, C₃-C₆ cycloalkyl, optionally substituted by -OH or -CH₂-OH; and tetrahydrofuranyl, tetrahydropyranyl, piperidinyl, or morpholinyl; or
R represents C₄-C₆ cycloalkyl optionally substituted with one or more substituents selected from the group consisting of -OH and fluorine; or
R represents phenyl-(C₁-C₂ alkyl), optionally substituted by two C₁-C₄ alkoxy; adamantyl, or pyridyl;
or a pharmaceutically acceptable salt or stereoisomer thereof or a pharmaceutically acceptable salt of the stereoisomer.

3. A compound of formula I according to claim 1 or 2, wherein
R represents a group of formula II
wherein
R2 and R3 represent independently from each other H, halogen, cyano, methyl, ethyl, propyl, isopropyl, *tert*-butyl, butyl, acetyl, trifluoromethyl, methoxy, trifluoromethoxy, methylthio, methylsulfinyl, methylsulfonyl, -COOH, -CH₂-COOH, dimethylamino; or -CO-NRxRy, wherein
Rx represents C₃-C₆ cycloalkyl or morpholinyl-ethyl, and Ry represents H, or
Rx and Ry form together with the nitrogen attached a pyrrolidinyl, a pyperidinyl or a morpholinyl ring optionally substituted with two methyl or one ethoxy-carbonyl; or
R represents C₁-C₆ alkyl optionally substituted with one substituent selected from the group consisting of -OH, -CF₃, C₃-C₆ cycloalkyl, optionally substituted by -OH or -CH₂-OH; tetrahydrofuranyl and tetrahydropyranyl; or
R represents cyclobutyl substituted with one or two fluorine; or
R represents phenyl-(C₁-C₂ alkyl) or pyridyl;
or a pharmaceutically acceptable salt or stereoisomer thereof or a pharmaceutically acceptable salt of the stereoisomer.

4. A compound of formula I according to claim 1, wherein
R represents a group of formula II
wherein
R2 and R3 represent independently from each other H, halogen, cyano, C₁-C₄ alkyl, acetyl, trifluoromethyl, methoxy, trifluoromethoxy, methylthio, methylsulfinyl, methylsulfonyl, -COOH , -CH₂-COOH, dimethylamino; or -CO-NRxRy, wherein
Rx represents C₃-C₆ cycloalkyl or morpholinyl-ethyl, and Ry represents H, or
Rx and Ry form together with the nitrogen attached a pyrrolidinyl, a pyperidinyl or a morpholinyl ring optionally substituted with one or two substituents selected from the group consisting of methyl and ethoxy-carbonyl,
or a pharmaceutically acceptable salt or stereoisomer thereof or a pharmaceutically acceptable salt of the stereoisomer.

5. A compound of formula I according to claim 4, wherein
wherein
R2 and R3 represent independently from each other H, halogen, cyano, methyl, ethyl, propyl, isopropyl, tertbutyl, butyl, acetyl, trifluoromethyl, methoxy, trifluoromethoxy, methylthio, methylsulfinyl, methylsulfonyl, -COOH, -CH₂-COOH, dimethylamino; or -CO-NRxRy, wherein Rx represents C₃-C₆ cycloalkyl or morpholinyl-ethyl, and Ry represents H, or
Rx and Ry form together with the nitrogen attached a pyrrolidinyl, a pyperidinyl or a morpholinyl ring optionally substituted with two methyl or one ethoxy-carbonyl,
or a pharmaceutically acceptable salt or stereoisomer thereof or a pharmaceutically acceptable salt of the stereoisomer.

6. A compound of formula I according to claim 1, wherein
R represents C₁-C₆ alkyl optionally substituted with one or more substituents selected from the group consisting of -OH, -COOH, -COO-(C₁-C₂ alkyl), -NH-CO-CF₃, -CF₃, C₃-C₆ cycloalkyl, optionally substituted by -OH or -CH₂-OH; tetrahydrofuranyl, tertahydropyranyl, morpholinyl and piperidinyl,
or a pharmaceutically acceptable salt or stereoisomer thereof or a pharmaceutically acceptable salt of the stereoisomer.

7. A compound of formula I according to claim 6, wherein
R represents C₁-C₆ alkyl optionally substituted with one substituent selected from the group consisting of -OH, -CF₃, C₃-C₆ cycloalkyl, optionally substituted by -OH or -CH₂-OH; tetrahydrofuranyl and tertahydropyranyl;
or a pharmaceutically acceptable salt or stereoisomer thereof or a pharmaceutically acceptable salt of the stereoisomer.

8. A compound of formula I according to claim 7, wherein
R represents C₁-C₆ alkyl optionally substituted with one substituent selected from the group consisting of -OH and C₃-C₆ cycloalkyl, optionally substituted by -OH or -CH₂-OH;
or a pharmaceutically acceptable salt or stereoisomer thereof or a pharmaceutically acceptable salt of the stereoisomer.

9. A compound of formula I according to claim 8, wherein
R represents C₁-C₄ alkyl substituted with one substituent selected from the group consisting of - OH and C₃-C₆ cycloalkyl substituted with -OH or -CH₂-OH;
or a pharmaceutically acceptable salt or stereoisomer thereof or a pharmaceutically acceptable salt of the stereoisomer.

10. A compound of formula I according to claim 1, wherein
R represents C₄-C₆ cycloalkyl optionally substituted with one or more substituents selected from the group consisting of -OH and fluorine,
or a pharmaceutically acceptable salt or stereoisomer thereof or a pharmaceutically acceptable salt of the stereoisomer.

11. A compound of formula I according to claim 10, wherein
R represents C₄ cycloalkyl substituted with one or two fluorine.
or a pharmaceutically acceptable salt or stereoisomer thereof or a pharmaceutically acceptable salt of the stereoisomer.

12. A compound of formula I according to claim 1, wherein
R represents phenyl-(C₁-C₂ alkyl), optionally substituted by one or two C₁-C₄ alkoxy; adamantyl, or pyridyl
or a pharmaceutically acceptable salt or stereoisomer thereof or a pharmaceutically acceptable salt of the stereoisomer.

13. A compound of formula I according to claim 12, wherein
R represents phenyl-(C₁-C₂ alkyl) or pyridyl
or a pharmaceutically acceptable salt or stereoisomer thereof or a pharmaceutically acceptable salt of the stereoisomer.

14. A compound according to any of claims 1-13 in its Z isomeric form.

15. A compound of formula I according to any of claims 1-3 selected from the group consisting of:
3-Phenyl-5-[1-pyridin-4-yl-meth-(*Z*)-ylidene]-imidazolidine-2,4-dione
3-((4-Chloro-phenyl)-5-[1-pyridin-4-yl-meth-(*Z*)-ylidene]-imidazolidine-2,4-dione
5-[1-Pyridin-4-yl-meth-(*Z*)-ylidene]-3-m-tolyl-imidazolidine-2,4-dione
3-((4-Ethyl-phenyl)-5-[1-pyridin-4-yl-meth-(*Z*)-ylidene]-imidazolidine-2,4-dione
3-((3-Chloro-phenyl)-5-[1-pyridin-4-yl-meth-(*Z*)-ylidene]-imidazolidine-2,4-dione
3-((3,4-Dichloro-phenyl)-5-[1-pyridin-4-yl-meth-(*Z*)-ylidene]-imidazolidine-2,4-dione
3-Benzyl-5-[1-pyridin-4-yl-meth-(*Z*)-ylidene]-imidazolidine-2,4-dione
3-((4-Methylsulfanyl-phenyl)-5-[1-pyridin-4-yl-meth-(*Z*)-ylidene]-imidazolidine-2,4-dione
3-Cyclohexyl-5-[1-pyridin-4-yl-meth-(*Z*)-ylidene]-imidazolidine-2,4-dione
3-((3,4-Difluoro-phenyl)-5-[1-pyridin-4-yl-meth-(*Z*)-ylidene]-imidazolidine-2,4-dione
4-{2,5-Dioxo-4-[1-pyridin-4-yl-meth-(*Z*)-ylidene]-imidazolidin-1-yl}-benzonitrile
3-((4-Fluoro-phenyl)-5-[1-pyridin-4-yl-meth-(*Z*)-ylidene]-imidazolidine-2,4-dione
3 -((3 -Acetyl-phenyl)-5 - [1-pyridin-4-yl-meth-(*Z*)-ylidene]-imidazolidine-2,4-dione
3-((4-Dimethylamino-phenyl)-5-[1-pyridin-4-yl-meth-(*Z*)-ylidene]-imidazolidine-2,4-dione
4-{2,5-Dioxo-4-[1-pyridin-4-yl-meth-(*Z*)-ylidene]-imidazolidin-1-yl}-benzoic acid
5-[1-Pyridin-4-yl-meth-(*Z*)-ylidene]-3-(3-trifluoromethyl-phenyl)-imidazolidine-2,4-dione
3-((4-Methoxy-phenyl)-5-[1-pyndin-4-yl-meth-(*Z*)-ylidene]-imidazolidine-2,4-dione
3-Phenethyl-5-[1-pyridin-4-yl-meth-(*Z*)-ylidene]-imidazolidine-2,4-dione
5-[1-Pyridin-4-yl-meth-(*Z*)-ylidene]-3-p-tolyl-imidazolidine-2,4-dione
3-((4-Bromo-phenyl)-5-[1-pyridin-4-yl-meth-(*Z*)-ylidene]-imidazolidine-2,4-dione
3-Isopropyl-5-[1-pyridin-4-yl-meth-(*Z*)-ylidene]-imidazolidine-2,4-dione
3-Hexy1-5-[1-pyridin-4-yl-meth-(*Z*)-ylidene]-imidazolidine-2,4-dione
3-((4-Acetyl-phenyl)-5-[1-pyridin-4-yl-meth-(*Z*)-ylidene]-imidazolidine-2,4-dione
3 -((4-Butyl-phenyl)-5 - [1-pyridin-4-yl-meth-(*Z*)-ylidene]-imidazolidine-2,4-dione
3-((4-Methanesulfinyl-phenyl)-5-[1-pyridin-4-yl-meth-(*Z*)-ylidene]-imidazolidine-2,4-dione
3-((4-Methanesulfonyl-phenyl)-5-[1-pyridin-4-yl-meth-(*Z*)-ylidene]-imidazolidine-2,4-dione
3-{2,5-Dioxo-4-[1-pyridin-4-yl-meth-(*Z*)-ylidene]-imidazolidin-1-yl}-benzoic acid
3-[3-(Morpholine-4-carbonyl)-phenyl]-5-[1-pyridin-4-y1-meth-(*Z*)-ylidene]-imidazolidine-2,4-dione
3-[4-(Morpholine-4-carbonyl)-phenyl]-5-[1-pyridin-4-yl-meth-(*Z*)-ylidene]-imidazolidine-2,4-dione
5-[1-Pyridin-4-yl-meth-(*Z*)-ylidene]-3-(4-trifluoromethoxy-phenyl)-imidazolidine-2,4-dione
5-[1-Pyridin-4-yl-meth-(*Z*)-ylidene]-3-(4-trifluoromethyl-phenyl)-imidazolidine-2,4-dione
(4-{2,5-Dioxo-4-[1-pyridin-4-yl-meth-(*Z*)-ylidene]-imidazolidin-1-yl}-phenyl)-acetic acid
3-((4-Iodo-phenyl)-5-[1-pyridin-4-yl-meth-(*Z*)-ylidene]-imidazolidine-2,4-dione
3-{2,5-Dioxo-4-[1-pyridin-4-yl-meth-(*Z*)-ylidene]-imidazolidin-1-yl}-N-(2-morpholin-4-yl-ethyl)-benzamide
3-((4-tert-Butyl-phenyl)-5-[1-pyridin-4-yl-meth-(*Z*)-ylidene]-imidazolidine-2,4-dione
3-[2-(3,4-Diethoxy-phenyl)-ethyl]-5-[1-pyridin-4-yl-meth-(*Z*)-ylidene]-imidazolidine-2,4-dione
5-[1-Pyridin-4-yl-meth-(*Z*)-ylidene]-3-(2-pyrrolidin-1-yl-ethyl)-imidazolidine-2,4-dione
3-Adamantan-1-yl-5-[1-pyridin-4-yl-meth-(*Z*)-ylidene]-imidazolidine-2,4-dione
3-Pyridin-3-yl-5-[1-pyridin-4-yl-meth-(*Z*)-ylidene]-imidazolidine-2,4-dione
3-((4-Hydroxy-cyclohexyl)-5-[1-pyridin-4-yl-meth-(*Z*)-ylidene]-imidazolidine-2,4-dione 3 -Propyl-5 - [1-pyridin-4-yl-meth-(*Z*)-ylidene]-imidazolidine-2,4-dione
3-*sec*-Butyl-5-[1-pyndin-4-yl-meth-(Z)-ylidene]-imidazolidine-2,4-dione
{2,5-Dioxo-4-[1-pyridin-4-yl-meth-(Z)-ylidene]-imidazolidin-1yl}-acetic acid ethyl ester
3-((3-Morpholin-4-yl-propyl)-5-[1-pyridin-4-yl-meth-(Z)-ylidene]-imidazolidine-2,4-dione
3-[4-((2R,6S)-2,6-Dimethyl-morpholine-4-carbonyl)-phenyl]-5-[1-pyridin-4-yl-meth-(Z)-ylidene]-imidazolidine-2,4-dione
{2,5-Dioxo-4-[1-pyridin-4-yl-meth-(Z)-ylidene]-imidazolidin-1-yl}-acetic acid 3-[4-(4-Methyl-piperidine-1-carbonyl)-phenyl]-5-[1-pyridin-4-yl-meth-(Z)-ylidene]-imidazolidine-2,4-dione
5-[1-Pyridin-4-yl-meth-(Z)-ylidene]-3-[4-(pyrrolidine-1-carbonyl)-phenyl]-imidazolidine-2,4-dione
N-Cyclopentyl-4-{2,5-dioxo-4-[1-pyridin-4-yl-meth-(Z)-ylidene]-imidazolidin-1-yl}-benzamide
N-Cyclopropyl-4-{2,5-dioxo-4-[1-pyridin-4-yl-meth-(Z)-ylidene]-imidazolidin-1-yl}-benzamide
N-Cyclohexyl-4-{2,5-dioxo-4-[1-pyridin-4-yl-meth-(Z)-ylidene]-imidazolidin-1-yl}-benzamide
3-((3-Hydroxy-2,2-dimethyl-propyl)-5-[1-pyridin-4-yl-meth-(Z)-ylidene]-imidazolidine-2,4-dione
3-((3,3-Difluoro-cyclobutyl)-5-[1-pyridin-4-yl-meth-(Z)-ylidene]-imidazolidine-2,4-dione
1-((4-{2,5-Dioxo-4-[1-pyridin-4-yl-meth-(Z)-ylidene]-imidazolidin-1-yl}-benzoyl)-piperidine-4-carboxylic acid ethyl ester
3-((1-Hydroxy-cyclohexylmethyl)-5-[1-pyridin-4-yl-meth-(Z)-ylidene]-imidazolidine-2,4-dione
5-[1-Pyridin-4-yl-meth-(Z)-ylidene]-3-(tetrahydro-pyran-4-ylmethyl)-imidazolidine-2,4-dione
3-((3-Hydroxy-3-methyl-butyl)-5-[1-pyridin-4-yl-meth-(Z)-ylidene]-imidazolidine-2,4-dione
3-((1-Hydroxy-cyclopentylmethyl)-5-[1-pyridin-4-yl-meth-(Z)-ylidene]-imidazolidine-2,4-dione
3-((1-Hydroxy-cyclobutylmethyl)-5-[1-pyridin-4-yl-meth-(Z)-ylidene]-imidazolidine-2,4-dione
5-[1-Pyridin-4-yl-meth-(Z)-ylidene]-3-(tetrahydro-furan-2-ylmethyl)-imidazolidine-2,4-dione
3-((2-Hydroxy-2-methyl-propyl)-5-[1-pyridin-4-yl-meth-(Z)-ylidene]-imidazolidine-2,4-dione
3-((*trans*-4-Hydroxy-cyclohexylmethyl)-5-[1-pyridin-4-yl-meth-(Z)-ylidene]-imidazolidine-2,4-dione
5-[1-Pyridin-4-yl-meth-(Z)-ylidene]-3-(2,2,2-trifluoro-ethyl)-imidazolidine-2,4-dione
3-((1-Hydroxymethyl-cyclobutylmethyl)-5-[1-pyridin-4-yl-meth-(Z)-ylidene]-imidazolidine-2,4-dione
3-((1-Hydroxymethyl-cyclopentylmethyl)-5-[1-pyridin-4-yl-meth-(Z)-ylidene]-imidazolidine-2,4-dione
N-(2-{2,5-Dioxo-4-[1-pyridin-4-yl-meth-(Z)-ylidene]-imidazolidin-1-yl}-ethyl)-2,2,2-trifluoroacetamide
or a pharmaceutically acceptable salt, stereoisomer or a pharmaceutically acceptable salt of the stereoisomer thereof.

16. A compound according to any of claims 1-15 or a pharmaceutically acceptable salt or stereoisomer thereof or a pharmaceutically acceptable salt of the stereoisomer for use in the treatment or prophylaxis of inflammatory diseases.

17. A compound according to claim 16 for use in the treatment or prophylaxis of inflammatory diseases ,wherein the inflammatory diseases are selected from COPD,IBD asthma, allergy, obesity, metabolic syndrome, diabetic nephropathy, cardiac hypertrophy and cerebral ischemia.

18. Pharmaceutical composition containing at least one compound according to any of claims 1-15 or a pharmaceutically acceptable salt or stereoisomer thereof or a pharmaceutically acceptable salt of the stereoisomer and at least one pharmaceutically acceptable excipient.
